# EUROPEAN PATENT APPLICATION

(11) **EP 4 585 690 A1**
(43) Date of publication of application: **16.07.2025**
(21) Application number: 23863511.4
(22) Date of filing: 06.09.2023
(51) Int. Cl.: C12N 15/63, C12N 15/113

(54) **SELF-CIRCULARIZATION RNA STRUCTURE**

(30) Priority: 06.09.2022 KR 20220112832
(71) Applicant: RZNOMICS INC., Yongin-si, Gyeonggi-do 16890 (KR)
(72) Inventor: LEE, Seong Wook, Seoul 02780 (KR); LEE, Kyung Hyun, Seongnam-si, Gyeonggi-do 13624 (KR); KIM, Seong Cheol, Seongnam-si, Gyeonggi-do 13505 (KR)
(74) Representative: De Clercq & Partners
(86) International application number: PCT/KR2023/013375
(87) International publication number: WO 2024/054047

(57) **Abstract**

A self-circularized RNA structure of the present invention can be expressed in a DNA vector and, at the same time, form circRNA by being circularized through a self-targeting and splicing reaction, wherein the circRNA consists of only a gene of interest. The gene of interest includes an IRES region, an initiation codon and a termination codon, and thus enables the rapid expression of a peptide and a protein.

## Description

### TECHNICAL FIELD

The following description relates to a self-circularization RNA construct with improved circularization efficiency.

The present disclosure stems from a project of the National Research Foundation of Korea named "Core Technology Development for Next Generation Vaccines for Infectious Diseases" (Project No. 2022M3E5F1017657) and was supported with funding by the Ministry of Science and ICT.

### BACKGROUND ART

Circular RNAs (circRNAs, cRNAs) are single-stranded transcripts covalently linked. Through RNA-seq data and a newly developed bioinformatics approach, more than tens of thousands of circRNA types have been identified in various living organisms. In eukaryotes, it is known that a circRNA may be generated through back-splicing from mRNA and may regulate gene expression by performing a microRNA sponge function in vivo. It is not known whether a circRNA induces immunogenicity, but it exists very stably in vivo due to its structural characteristics.

Recently, the development of therapeutic agents using messenger RNA (mRNA) has been active. However, mRNA has a limitation in that it easily degrades in vivo and has a relatively short half-life. In order to overcome this limitation, studies such as studies on attaching poly(A) tail to mRNA to improve stability are in progress. In the same vein, US 10,953,033 discloses a circRNA for the purpose of gene expression in vivo based on the structural characteristics of the circRNA.

### DISCLOSURE OF THE INVENTION

### TECHNICAL GOALS

A technical task of the present disclosure is to provide an RNA construct that is circularized by performing a self-targeting and splicing reaction.

However, the technical task of the present disclosure is not limited to that described above, and other tasks not mentioned herein will be clearly understood by one of ordinary skill in the art from the following description.

### TECHNICAL SOLUTIONS

In order to achieve the above mentioned goals, the present disclosure provides a self-circularization RNA construct. The RNA construct of the present disclosure has a structure of 5' - IGS (internal guide sequence) - ribozyme - gene of interest - target site - 3', and may form a P1 helix including a bulge.

A P1 helix is a helix structure formed through a complementary binding between the nucleotide sequence linked to the front end (5' direction) of the ribozyme and the nucleotide sequence in the 3' direction of the transcript in which conjugation is induced by the ribozyme, when the secondary structure of the Group I intron ribozyme is being formed.

In one embodiment of the present disclosure, the IGS region may include or consist of a nucleotide sequence represented by 5'-GNNNNN-3', and the target site region may include or consist of a nucleotide sequence represented by 5'-N'N'N'N'N'U-3'. The IGS region and the target site may form a guanine (G): uracil (U) wobble base pair, and the **P1** helix may be formed through a complementary binding between the IGS region and the target site.

In another embodiment of the present disclosure, N of the IGS region and N' of the target site region may each independently be A, G, C, or U; however, preferably, the nucleotide to which the IGS region and the target site region may complementarily bind may be one or more nucleotide, and more preferably, N and N', excluding the wobble base pair, may be reverse complementary nucleotides.

In another embodiment of the present disclosure, the RNA structure may include a nucleotide sequence extending in the 5' direction of the IGS region and a nucleotide sequence extending in the 3' direction of the target site region, forming a bulge in the P1 helix.

In another embodiment of the present disclosure, the nucleotide sequences extending in the 5' direction of the IGS region and in the 3' direction of the target site region are designed not to bind complementarily to each other, and the number of bases extending in a given direction in each of said regions may be independently 1 to 10 nt.

In another embodiment of the present disclosure, the length of the extended nucleotide sequence forming the bulge may be 1 to 10 nt, preferably 2 to 5 nt, more preferably 3 to 5 nt.

In another embodiment of the present disclosure, the ribozyme may be a Group I intron ribozyme, and the ribozyme may include or consist of a nucleotide sequence represented by SEQ ID NO: 6.

In yet another embodiment of the present disclosure, the construct may include a nucleotide extending in a 5' direction of the IGS region such that a P1 helix and a P10 helix may be formed. In this connection, the P1 helix may be formed in a region in which the complementary binding between the IGS region and the target site is formed with a nucleotide extending in a 3' direction of the target site, and the P10 helix may be formed in a region in which complementary binding to a sequence reverse complementary to the extended nucleotide located between a ribozyme and a GOI region is formed with the nucleotide extending in the 5' direction of the IGS region. The length of the extended nucleotide forming the P1 helix may be 3-nt, and the length of the extended nucleotide forming the P10 helix may be 6-nt.

In yet another embodiment of the present disclosure, the construct may form a P1 helix but not a P10 helix.

In yet another embodiment of the present disclosure, the construct may include an antisense sequence (AS) and an antisense binding sequence (ABS) region capable of complementary binding to each other at the 5' end and the 3' end.

In yet another embodiment of the present disclosure, the ABS region may consist of a sequence reverse complementary to the AS region.

In yet another embodiment of the present disclosure, the length of the AS region may vary depending on GOI, but may be 10-nt to 500-nt, preferably more than 50-nt and less than 400-nt, and more preferably 150-nt to 350-nt.

In yet another embodiment of the present disclosure, the GOI region may include an internal ribosome entry site (IRES) region at the 5' end, and may include an initiation codon and a termination codon.

In yet another embodiment of the present disclosure, the construct may further include a spacer region including a random nucleotide sequence, in which the spacer region may be located between the IGS region and the gene of interest region and/or between the gene of interest region and the target site region.

In yet another embodiment of the present disclosure, the spacer region may include or consist of poly(A), and the poly(A) is a polynucleotide in which adenine (A) is repeatedly linked, in which the A may be linked repeatedly 10 to 50 times, and preferably linked repeatedly 30 times.

### EFFECTS

The self-circularized RNA construct of the present disclosure may be expressed in a DNA vector and simultaneously circularized through a self-targeting & splicing reaction without a separate GTP treatment to form a circRNA. The circRNA may consist only of a gene of interest, and the gene of interest has the advantage of being able to rapidly express a peptide or protein, including an IRES region, an initiation codon, and a termination codon. In addition, a circRNA has a circular structure and has a stable and long half-life because the 5' and 3' ends are not exposed. Accordingly, functional RNA such as miRNA, anti-miRNA, shRNA, aptamers, mRNA vaccines, mRNA therapeutic agents, antibodies, vaccine adjuvants, CAR-T mRNA, genomes or RNA editing guide RNA may be produced in the form of circRNA, ensuring high stability within cells.

### BRIEF DESCRIPTION OF DRAWINGS

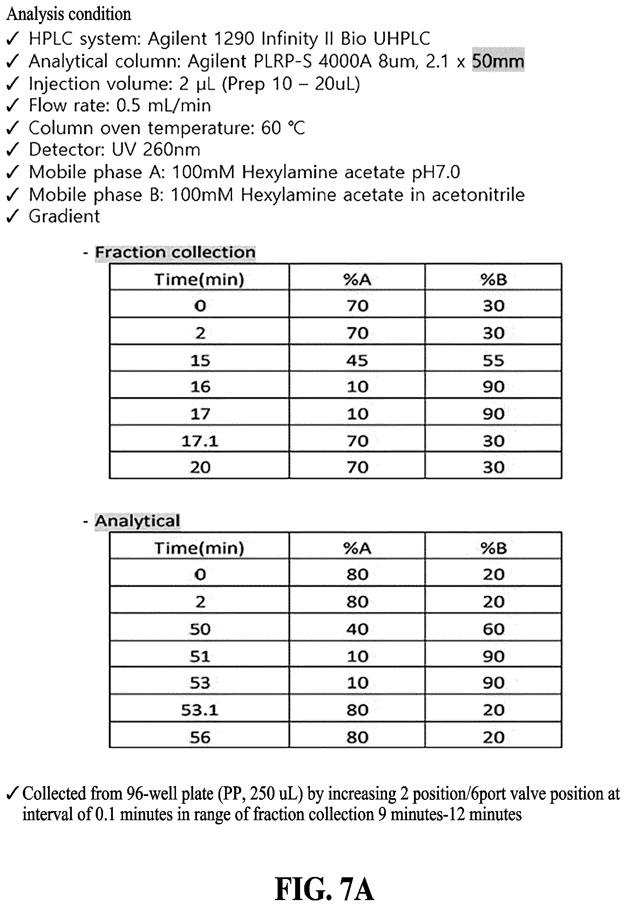

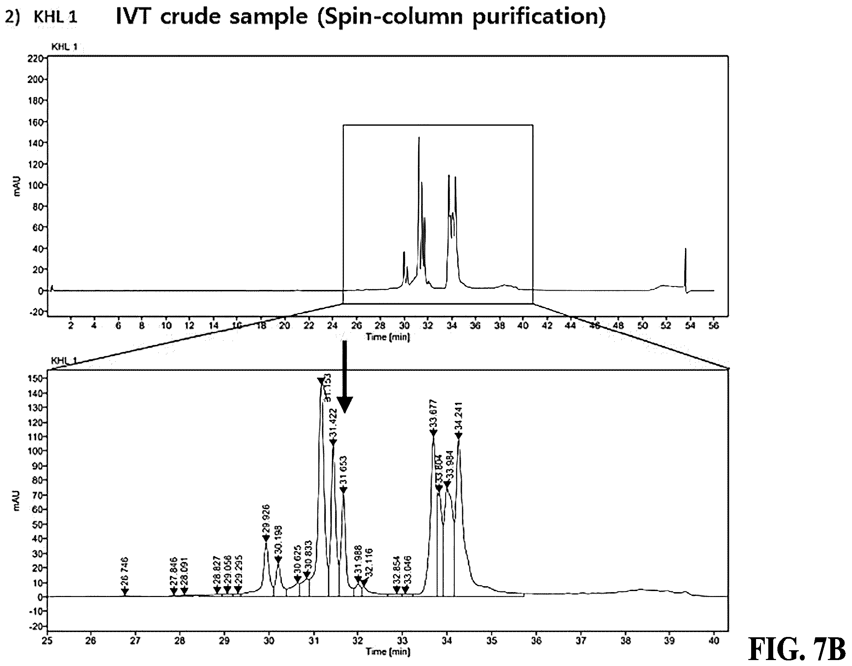

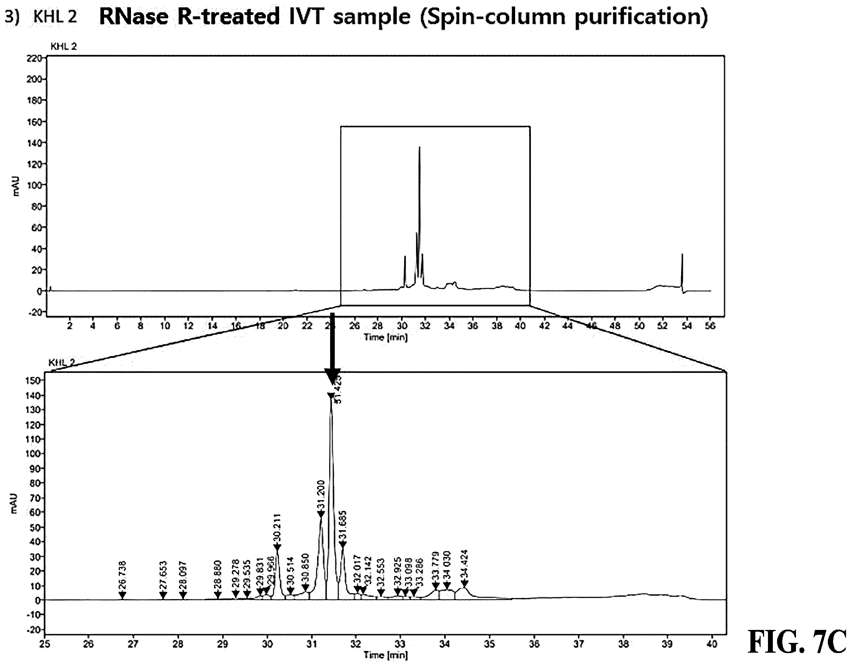

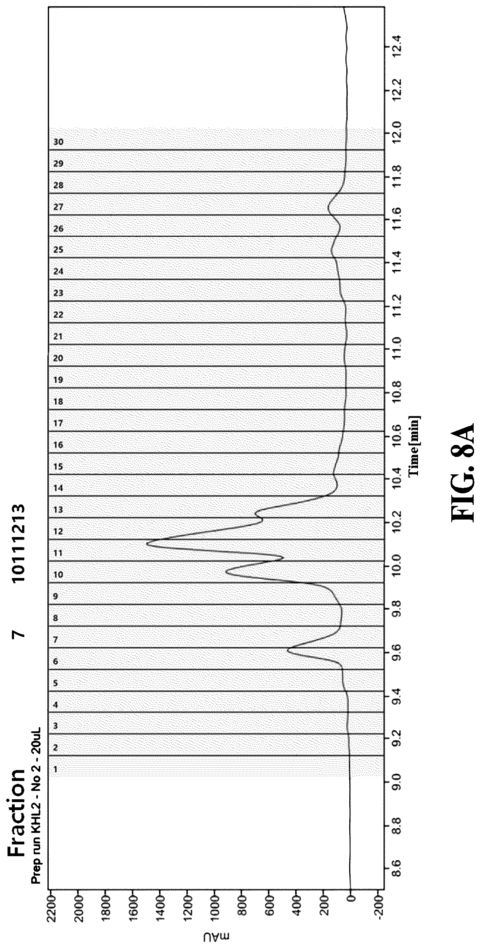

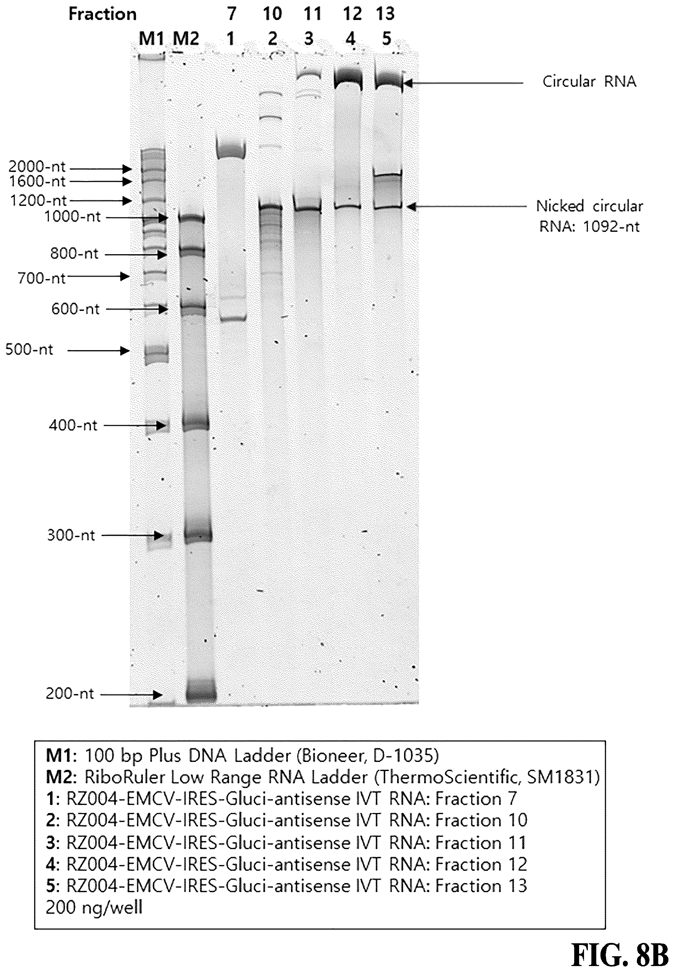

FIGS. 1A and 1B are schematic diagrams of a process in which a self-circularization RNA construct of an embodiment of the present disclosure including only IGS, ribozyme, gene of interest, and target site region is formed into a circRNA by a self-targeting and splicing (STS) reaction.
FIG. 2A is a schematic diagram of a process in which a self-circularization RNA construct of an embodiment of the present disclosure including AS, IGS, ribozyme, gene of interest, target site, and ABS region is formed into a circRNA by an STS reaction.
FIG. 2B is a schematic diagram of a self-circularization RNA construct of an embodiment of the present disclosure including IRES and a termination codon in the gene of interest region to enable transgene translation, and further including nucleotides extending in a 5' direction such that a P1 helix and P10 helix may be formed, and a process in which the construct is formed into a circRNA by an STS reaction.
FIG. 2C shows one form of a self-circularization RNA construct of an embodiment of the present disclosure.
FIG. 2D shows a DNA template nucleotide sequence for producing a self-circularization RNA construct expression vector of an embodiment of the present disclosure.

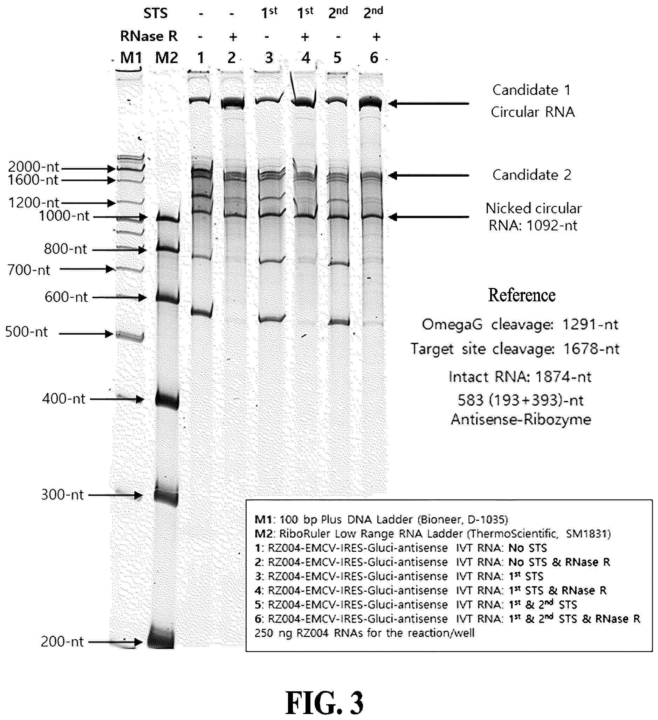

FIG. 3 shows the results of electrophoresis performed on a polyacrylamide gel to identify whether a circRNA is generated immediately after in vitro transcription of the self-circularization RNA construct expression vector of an embodiment of the present disclosure, without additional GTP treatment. Samples obtained immediately after transcription (direct STS) and samples obtained after the first and second STS reaction stages in which additional GTP treatment is performed to induce primary and secondary circularization reactions were used, and a portion of each sample was treated with RNase R to remove linear RNA, and circRNA in the samples was concentrated and electrophoresed.
FIG. 4 verifies that RNA assumed to be circRNA of the Candidate 1 band identified from the results of FIG. 3, is circRNA. FIG. 4A shows a result of extracting RNA from the Candidate 1 band and performing RT-PCR. FIG. 4B shows the sequencing result of RNA extracted from the Candidate 1 band.
FIG. 5 verifies that the RNA of the Candidate 1 band identified from the results of FIG. 4 is a monomer, and shows the results of electrophoresis after RNA assumed to be circRNA was extracted from the Candidate 1 and 2 bands identified from the results of FIG. 3 and inducing nicks by treatment with Mg²⁺.
FIG. 6 shows a result of identifying whether the self-circularization RNA construct expression vector of an embodiment of the present disclosure generates a circRNA in cells and whether the transgene included in the circRNA is expressed. Specifically, FIG. 6A shows a structure of the self-circularization RNA construct expression vector, FIG. 6B shows a result of identifying the expression of transgene through a luciferase activity assay, and FIGS. 6C and 6D show RT-PCR and sequencing results verifying that the transgene is expressed in circRNA.
FIGS. 7 and 8 show conditions for purifying circRNA by HPLC and results of purifying circRNA by HPLC.
Specifically, FIG. 7A shows HPLC analysis conditions using an Ultra HPLC system, FIG. 7B shows a result of column purification of a sample obtained immediately after in vitro transcription, and FIG. 7C shows a result of column purification after the sample was treated with RNase R. In addition, FIG. 8A shows a result of identifying a peak in the fraction obtained through column purification, and FIG. 8B shows an electrophoresis result of fractions 7, 10, 11, 12, and 13 in which peaks are prominent shown in FIG. 8A.

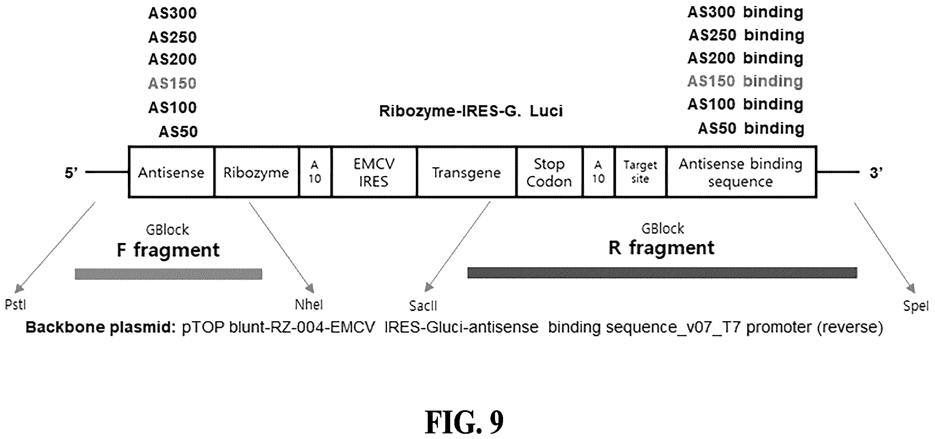

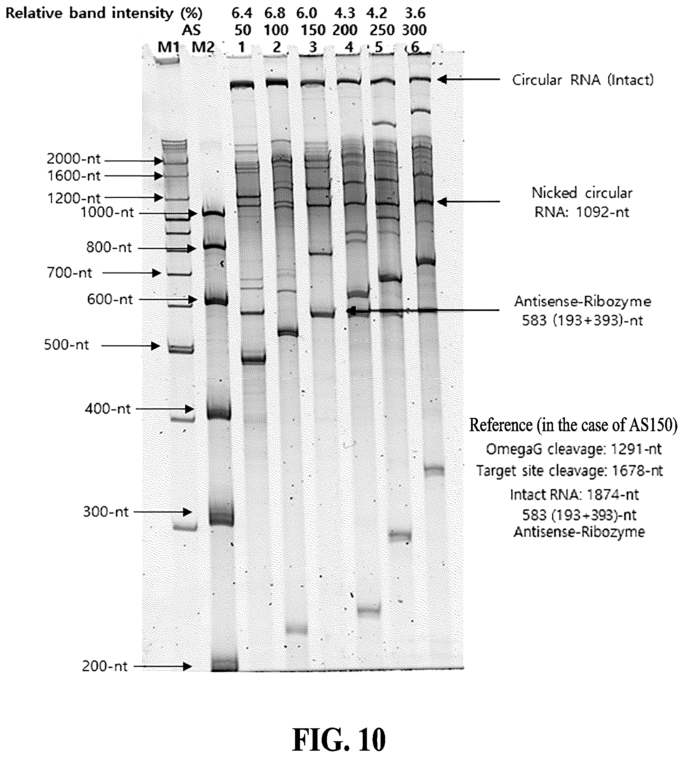

FIGS. 9 and 10 illustrate the effect of an AS region on STS reaction and circularization in a self-circularization RNA construct of an embodiment of the present disclosure. FIG. 9 shows a structure of a self-circularization RNA including AS regions of different lengths. FIG. 10 shows an electrophoresis result of a sample obtained after in vitro transcription of a vector expressing the RNA.

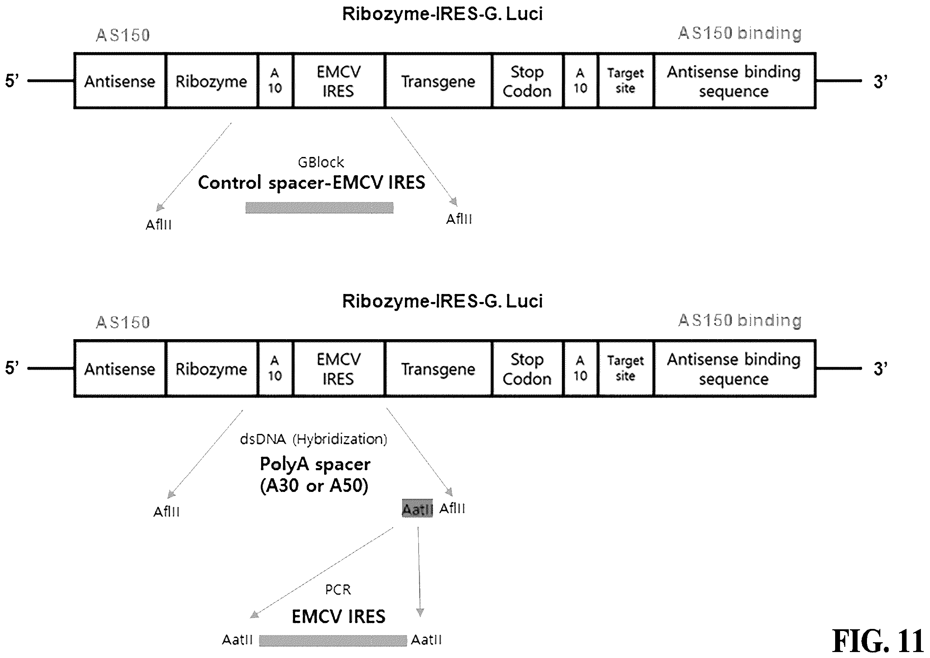

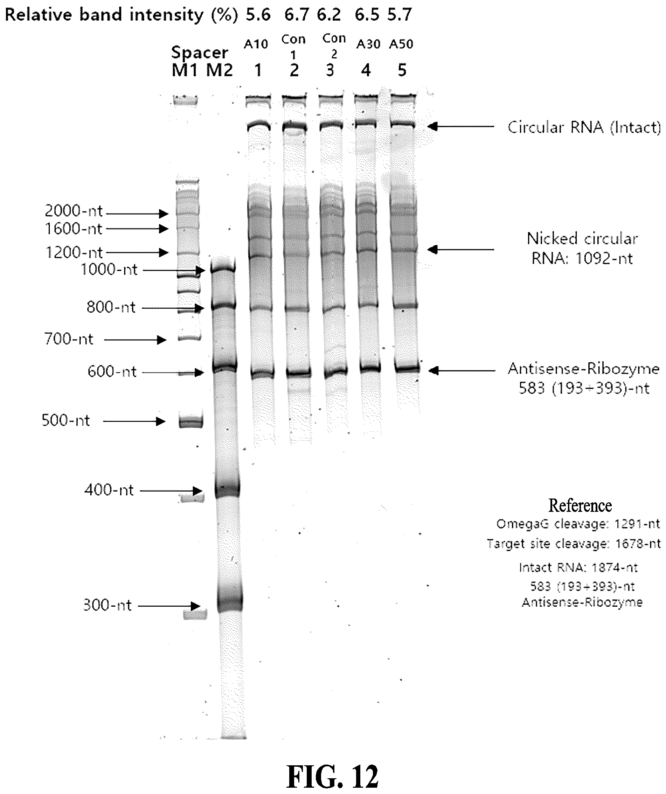

FIGS. 11 and 12 illustrate the effect of a spacer region on STS reaction and circularization in a self-circularization RNA construct of an embodiment of the present disclosure. FIG. 11 shows a structure of self-circularization RNA including a spacer of a control group (control spacer) and poly(A) spacers of various lengths. FIG. 12 shows an electrophoresis result of a sample obtained after in vitro transcription of a vector expressing the RNA.

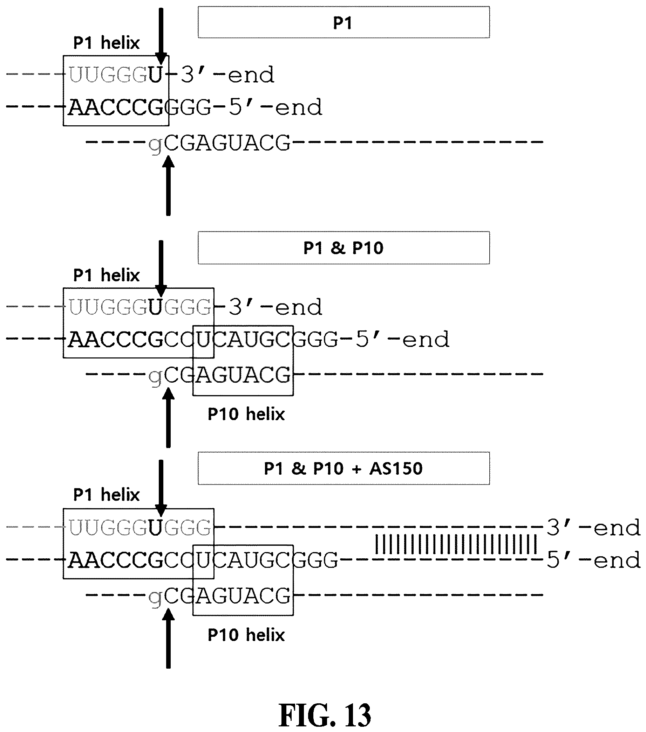

FIG. 13 indicates a nucleotide sequence near a region cleaved by a ribozyme and each region of: a self-circularization RNA including only a P1 region; self-circularization RNA including only P1 and P10 regions; and a self-circularization RNA including a P1 region, a P10 region, and an AS region.

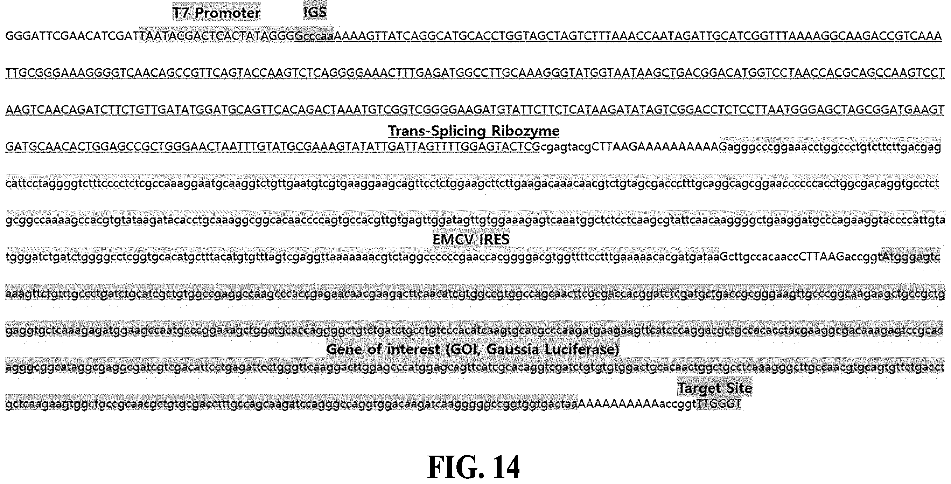

FIG. 14 shows a nucleotide sequence of a DNA template for preparing a self-circularization RNA expression vector including only a P1 region without P10 and AS regions.

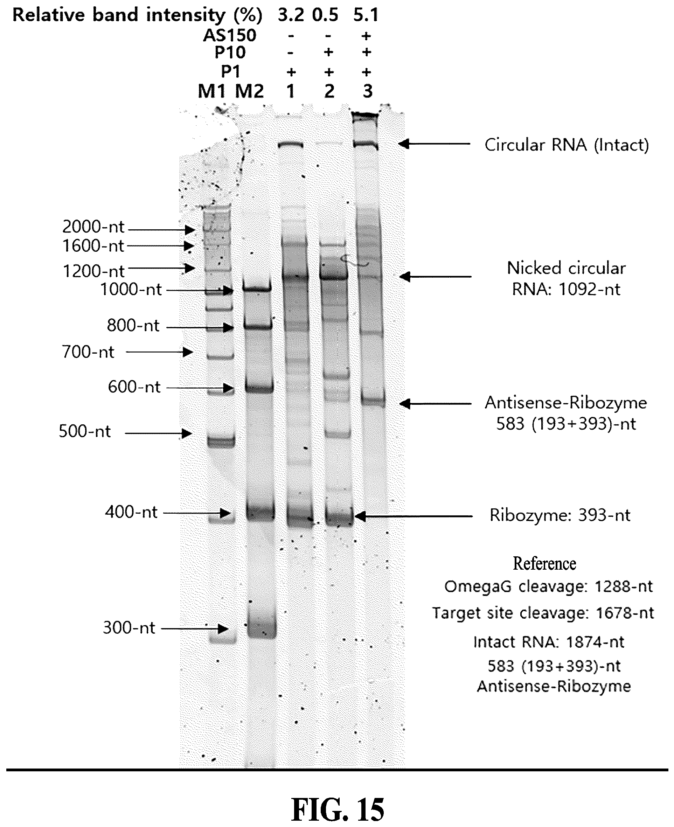

FIG. 15 shows an electrophoresis result of a sample obtained after in vitro transcription of each self-circularization RNA expression vector of FIG. 13.

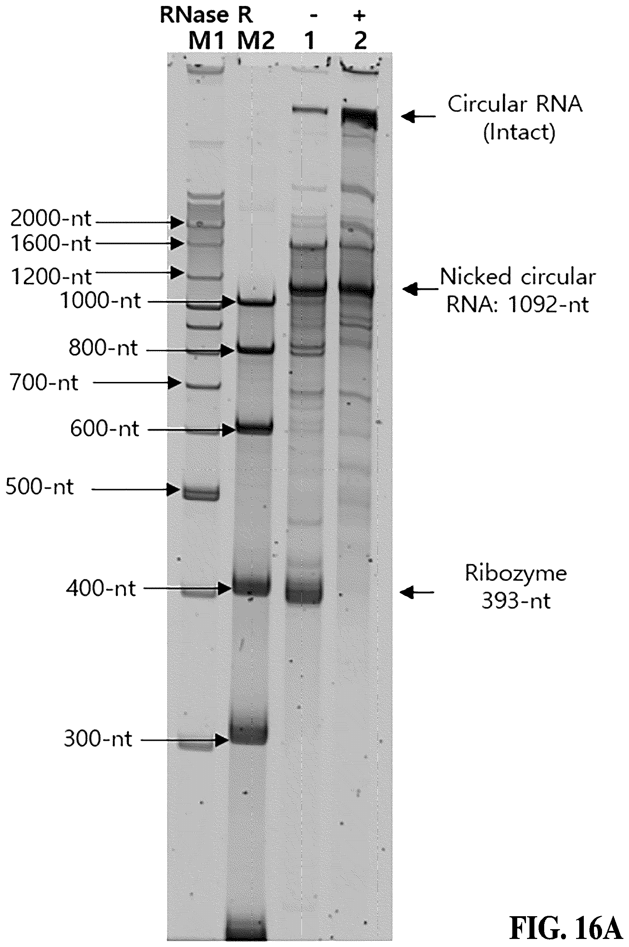

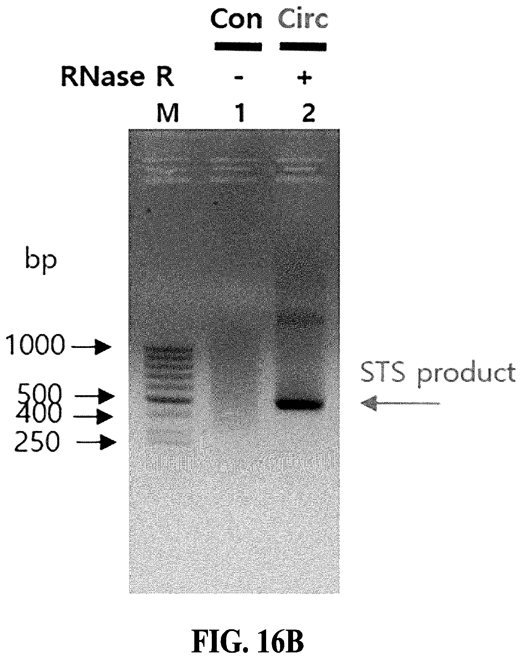

FIGS. 16A and 16B show the results of electrophoresis identifying that a self-circularization RNA construct is formed into a circRNA after in vitro transcription in a self-circularization RNA expression vector including only a P1 region without P10 and AS regions.

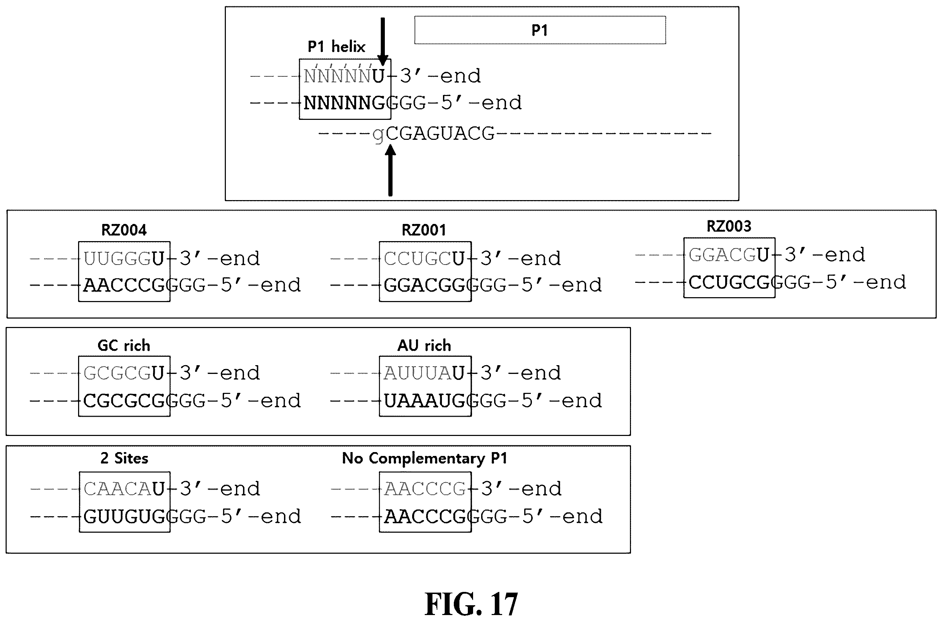

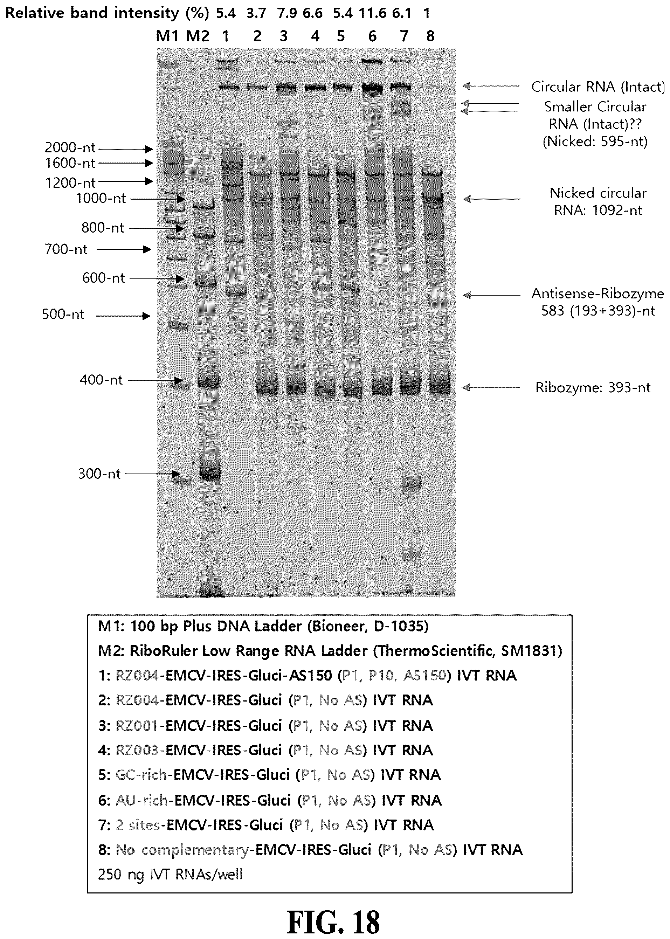

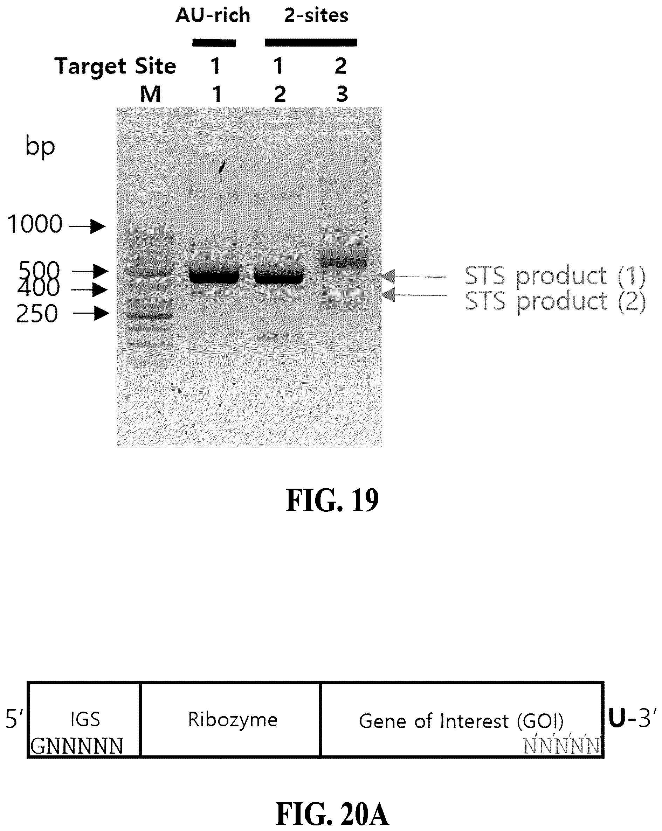

FIGS. 17 to 19 illustrate the effect of a nucleotide sequence of a P1 region on STS reaction and circularization in a self-circularization RNA construct of an embodiment of the present disclosure. Specifically, FIG. 17 shows a design of the P1 region of each different nucleotide sequence, FIG. 18 shows an electrophoresis result of a sample obtained after in vitro transcription of a self-circularization RNA expression vector having the P1 region of FIG. 17, and FIG. 19 shows an electrophoresis result of a sample obtained after in vitro transcription of a self-circularization RNA expression vector having an AU-rich P1 region and a self-circularization RNA expression vector having 2 sites for P1 formation, and shows an RT-PCR electrophoresis result identifying circular RNA.

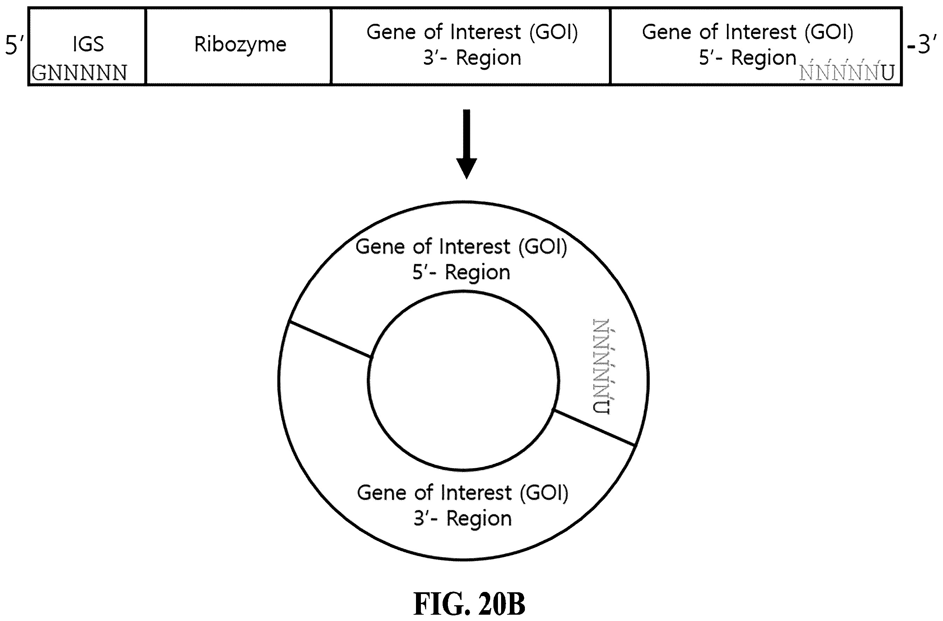

FIG. 20 is a simplified diagram of only the essential configuration of the self-circularization RNA construct. FIG. 20A shows that circRNA may be made from an RNA construct including only IGS, a ribozyme and GOI region, and a uracil base at the 3' end. FIG. 20B is a schematic diagram showing that circRNA may be made from simply IGS, a ribozyme, and RNA constructs of the GOI region when the uracil base is included at the 3' end of a GOI, in which the generated circRNA consists only of the GOI. In other words, FIG. 20B is a schematic diagram showing that when uracil is included after 5 unique nucleotide sequences at any site in the GOI, with that part as the 3' end, the rest of the 3' end of the GOI is sent to right behind the ribozyme, so that circular RNA consists only of the GOI without additional uracil.

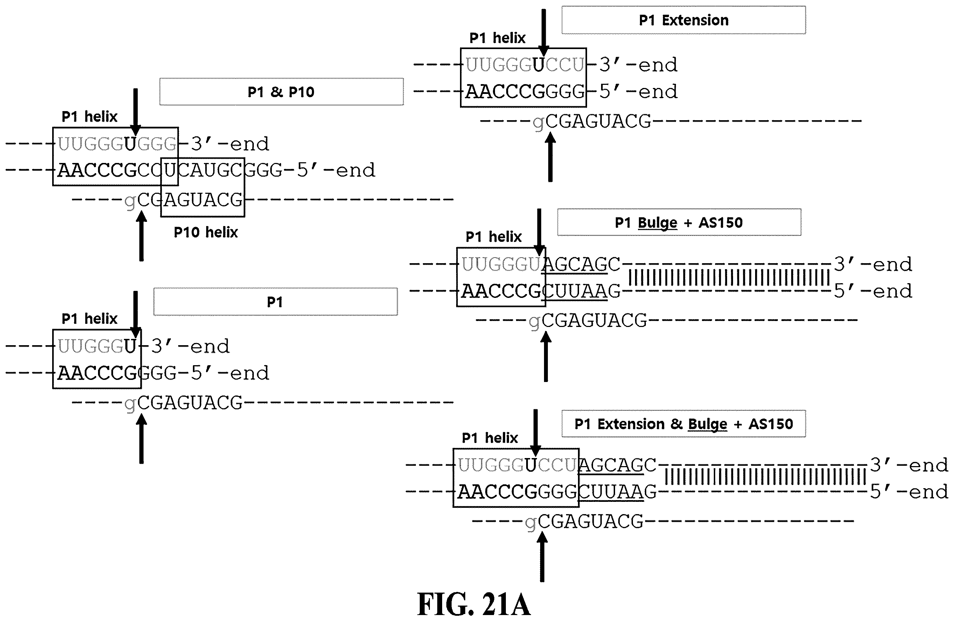

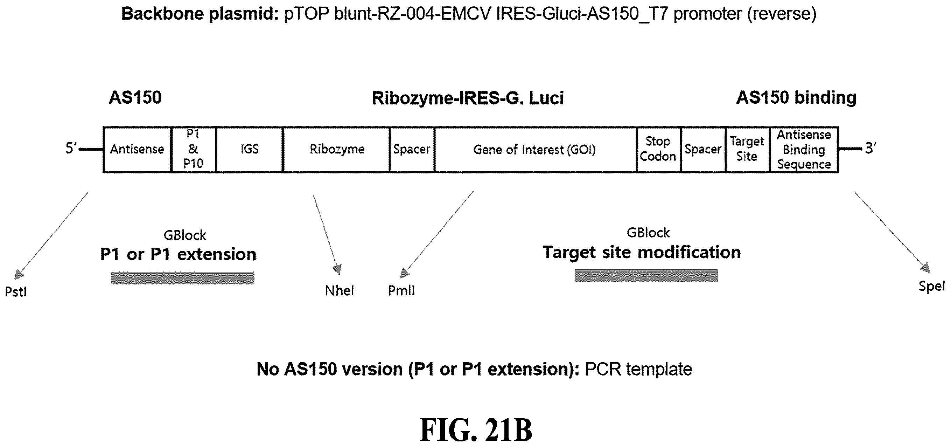

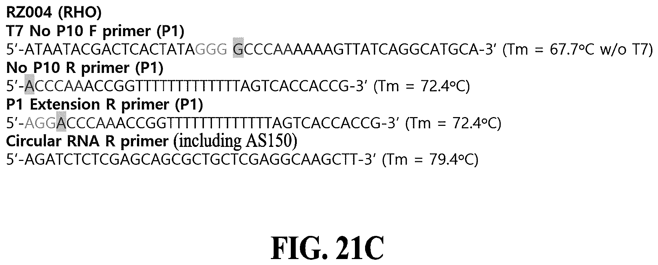

FIG. 21A is a diagram of various P1 helix variants designed by adding bases in the 5' direction from 5'-GNNNNN-3' in the IGS region; FIG. 21B is a schematic diagram of the structure of a self-circularization RNA construct expression vector containing the various P1 helix variants including a diagram of the production thereof; and FIG. 21C shows primers for amplification of the vectors. In FIG. 21A, the red arrows indicate the expected location of the STS junction.

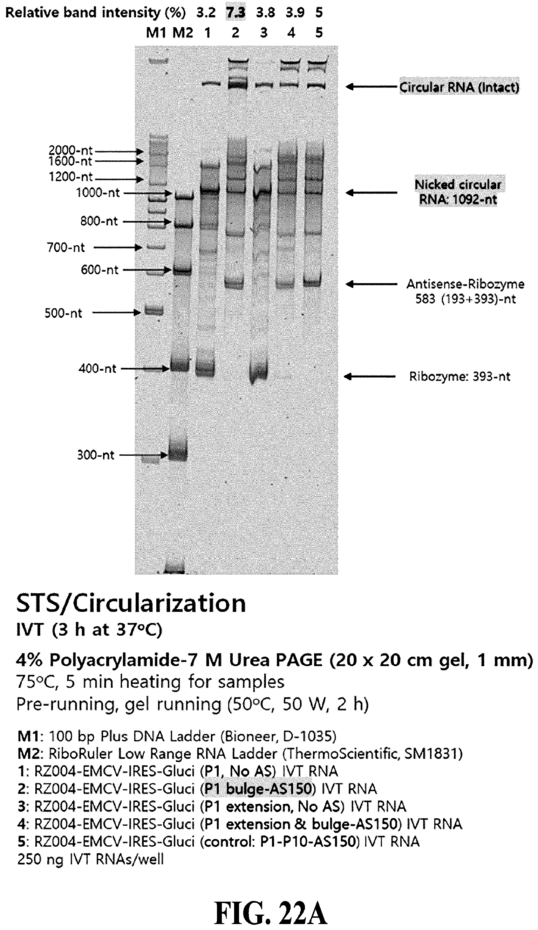

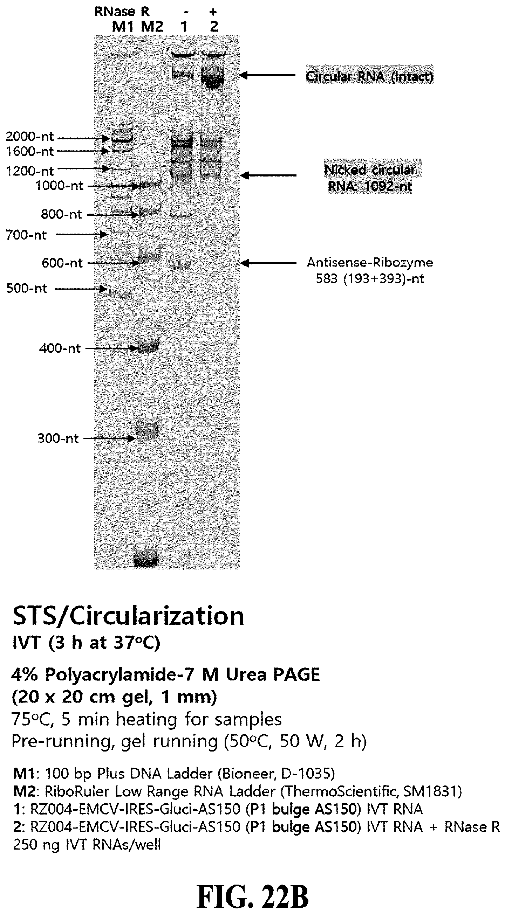

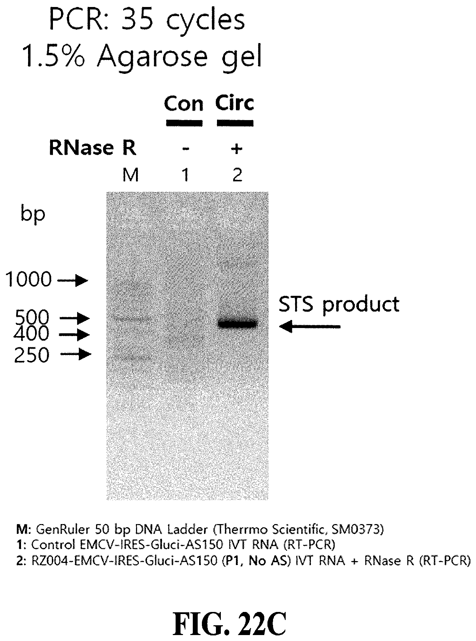

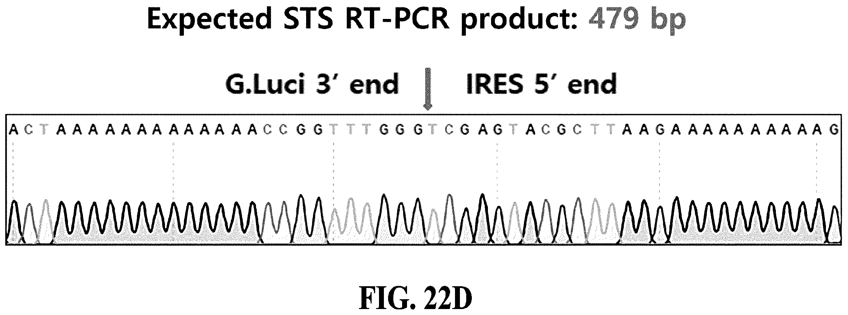

FIG. 22A shows the result of electrophoresis performed on the STS reaction product under IVT conditions of the self-circularization RNA construct expression vector containing the various P1 helix variants; FIG. 22B shows the result of performing RNase R treatment followed by electrophoresis on the STS reaction product under IVT conditions of the P1 bulge-AS construct expression vector, wherein the construct has the highest circular RNA production efficiency among the ones shown in FIG. 22A; FIG. 22C shows the result of performing RT-PCR on an RNA sample of the circRNA band shown in the result of electrophoresis after RNase R treatment; and FIG. 22D shows the result of sequencing the RNA extracted from the band.

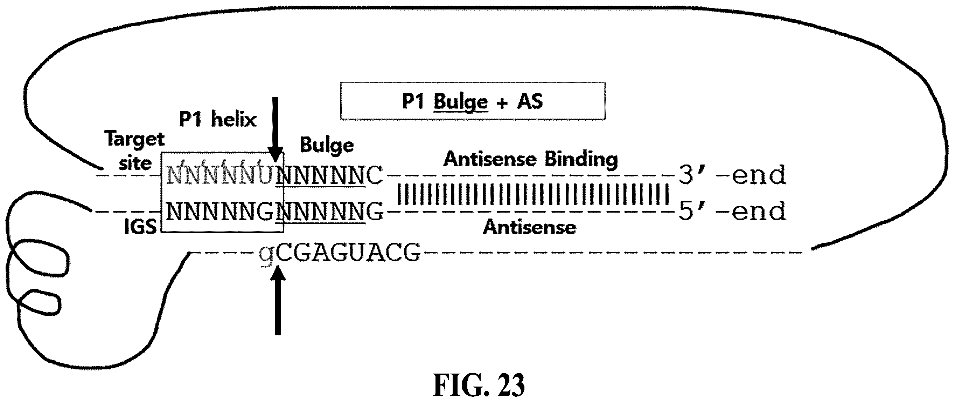

FIG. 23 is a schematic diagram of an example embodiment structure of the P1 bulge.

### BEST MODE FOR CARRYING OUT THE INVENTION

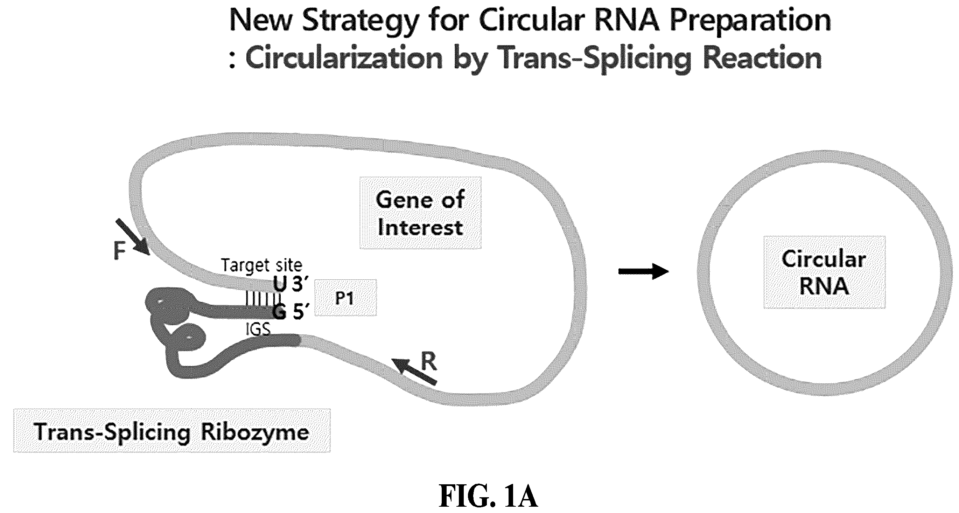

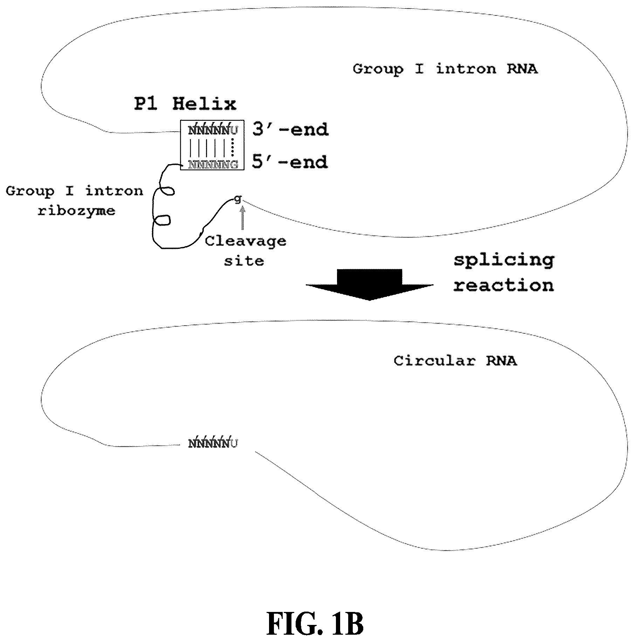

The present inventors used a trans-splicing ribozyme (T/S ribozyme) to prepare a circRNA, and devised a system in which the RNA containing a gene of interest performs a self-targeting and splicing reaction to be circularized (hereinafter referred to as "circularization system by self-targeting & splicing reaction") (FIGS. 1A and 1B).

A Group I intron ribozyme may induce trans-splicing by cleaving the target RNA through two successive trans-esterification reactions and then linking separate transcripts to each other at the cleaved 3' end.

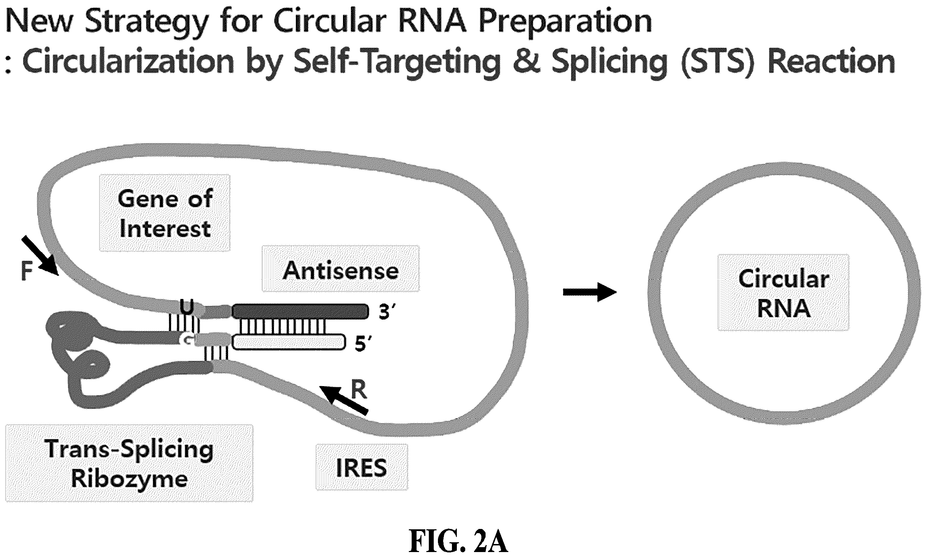

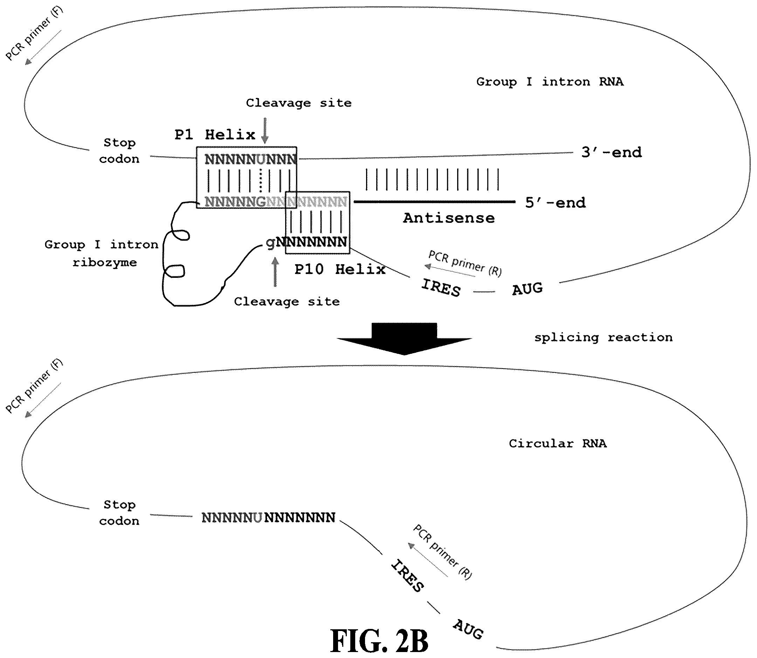

Accordingly, in the system of an embodiment of the present disclosure, an internal guide sequence (IGS) is configured in the 5' direction of a gene of interest (GOI) and a target site is configured in the 3' direction such that the IGS binds complementarily to the target site, and in the meantime, a guanine (G) : uracil (U) wobble base pair is formed, thereby inducing cleavage and splicing by the ribozyme located between the GOI and IGS so that a circRNA may be produced (FIGS. 2A and 2B).

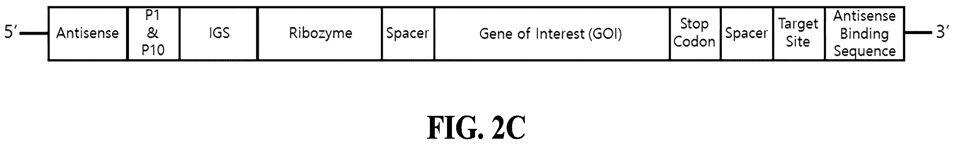

The present inventors focused on the previous study (Mol Ther. 2005 Nov; 12(5):824-34) for improving the trans-splicing efficiency of the Group I intron ribozyme, and as illustrated in FIG. 2C, designed a self-circularization RNA construct such that an AS (antisense sequence) region and an ABS (antisense binding sequence) capable of complementary binding to each other exist at the 5' and 3' ends of the self-circularization RNA construct, and P1 and P10 helixes are formed before/after the secondary structure of the ribozyme, and prepared a DNA template capable of expressing the RNA construct under a T7 promoter (Example 1).

Subsequently, a vector into which the DNA template was inserted was produced and transcribed in vitro (IVT), and then the formation of a circRNA was identified. As a result, it was found that the vector expresses a self-circularization RNA construct, and the RNA construct forms a monomeric circRNA through a self-targeting and splicing (STS) reaction immediately after transcription even without the addition of GTP (Example 2).

Furthermore, the present inventors tried to identify whether the self-circularization RNA construct expression vector may express the RNA construct of an embodiment of the present disclosure even in cells, and whether the expressed RNA may express a gene of interest loaded in the form of a circRNA. Specifically, a plasmid vector was produced by including IRES and a termination codon in a gene of interest to enable translation of the gene (transgene) in the cells and using gaussia luciferase as a transgene in the gene of interest, and the plasmid vector was transformed into cells to identify the production of a circRNA and luciferase activity. As a result, it was identified at the molecular level that the produced plasmid vector expressed the self-circularization RNA construct in the cells, the construct was circularized into a circRNA by a ribozyme, and the gene of interest was expressed in the circRNA (Example 3).

Subsequently, the present inventors attempted to optimize an RNA construct to efficiently form a circRNA through an immediate STS (direct STS) reaction by IVT.

First, the direct STS reaction rate according to a length of an AS region by IVT was identified through a specific experiment. Specifically, a self-circularization RNA expression vector was produced such that the lengths of the AS region and the ABS region were 50, 100, 150, 200, 250, or 300-nt, and the direct STS efficiency was identified after the vector was subjected to IVT. As a result, it was identified that the self-circularization efficiency was remarkably reduced at a length of 200-nt or more. Although the self-circularization efficiency was similar at 50, 100, and 150-nt lengths, it was identified that the in vitro transcription reaction itself decreased when the AS region and the ABS region of a 50-nt or 100-nt length were included. In terms of circRNA production efficiency, it was identified that the lengths of the AS region and the ABS region were preferably 150-nt (Example 5).

Like the circRNA production efficiency according to the lengths of the AS region and the ABS region was identified, the circRNA production efficiency according to the length and nucleotide sequence of a spacer region by IVT was identified. As a result, on the other hand, it was found that although its length and nucleotide sequence did not significantly affect the circRNA production efficiency, the linking of 30 adenines (A) was sufficient not to create a structural conflict that may occur due to a narrow gap between a ribozyme and the IRES in the case of the ribozyme and the EMCV IRES (Example 6).

In an embodiment of the present disclosure, a Group I intron ribozyme capable of a continuous trans-esterification reaction was used as a ribozyme. Group I intron ribozymes induce trans-splicing by linking separately existing transcripts to each other at the cleaved 3' end after cleavage of the target site, and link the 5' site of a GOI to the cleaved 3' end, which is not a separately existing transcript in a self-circularization RNA construct. The intention was to identify whether the P1 and P10 helix regions, known to increase trans-splicing efficiency, and the AS region and ABS region at both ends of the self-circularization RNA construct also had a positive effect on circularization efficiency.

Specifically, the present inventors produced a self-circularization RNA expression vector including only a P1 helix region, only P1 and P10 helix regions, or both the P1 and P10 helix regions and the AS region, and identified direct STS efficiency after IVT of the vector. As a result, surprisingly, it was found that the P10 helix region in the self-circularization RNA construct reduced the circRNA production efficiency when the P10 helix region was in the presence of the P1 helix region. Meanwhile, excellent circRNA production efficiency was exhibited even when only the P1 helix region was included without the AS region (Example 7).

Furthermore, the present inventors designed a self-circularization RNA construct such that only IGS forms the P1 helix, leaving only a gene of interest in the final product, circRNA, and identified whether an STS reaction occurs in various IGS sequences. As a result, surprisingly, even when only the IGS region formed the P1 helix, it was identified that the STS reaction of the self-circularization RNA construct expressed in DNA was induced, and furthermore, that the circRNA was formed even though the IGS region and the target site region were not complementary to each other. However, when the IGS region and the target site region are not complementary to each other, a non-specific reaction may occur at an undesired site, and thereby an unwanted product may be generated. The IGS region of 5'-GNNNNN-3' and the target site region of 5'-N'N'N'N'N'U-3' showed that the circRNA production efficiency increased as the ratio of nucleotide sequences capable of complementary binding to each other increased, with certain sequences showing higher circRNA production efficiency (Example 8).

The present inventors added bases in the 5' direction from the 5'-GNNNNN-3' of the IGS to produce various P1 helix variants and determined if the bases added in the 5' direction of the IGS improved the efficiency of circular RNA production. Specifically, constructs were designed in which the bases added in the 5' direction of the IGS formed an extension of the P1 helix (P1 extension) and a bulge of the P1 helix (P1 bulge), and the differences in circular RNA production efficiency of the P1 extension construct, the P1 bulge-AS150 construct, and the P1 extension & bulge-AS150 construct were compared with those of the P1 construct and the P1-P10 construct. The result showed that, unlike the extension of the P1 helix, the formation of the bulge improved self-circularization efficiency. Furthermore, the result showed that the P1 bulge-AS150 construct had a higher circular RNA production rate than the P1-P10-AS150 construct. From the above, it can be seen that the STS reaction by ribozyme was more effectively induced when a bulge (where RNA forms a secondary structure and thus appears to be puffed up) is present within the P1 helix (Example 9).

The P1 helix is a helix structure formed through a complementary bond between the nucleotide sequence linked to the front end (5' direction) of the ribozyme and the nucleotide sequence in the 3' direction of the transcript in which conjugation is induced by the ribozyme, in the formation of the secondary structure of the Group I intron ribozyme, and the P10 helix is a helix structure formed through a complementary bond between a front end region of the ribozyme and a nucleotide sequence in the 5' direction of the transcript cleaved by the ribozyme.

In an embodiment of the present disclosure, the P1 helix may be formed through a complementary bond between the IGS at the 5' end and the target site at the 3' end in the self-circularization RNA construct of an embodiment of the present disclosure, and the P10 helix may be formed through a complementary bond between a nucleotide sequence extended at the 5' end and a nucleotide sequence linked to the rear end (3' direction) of the ribozyme.

Herein, the nucleotide sequence forming the P1 helix is referred to by the term "P1 region" or "P1 helix region", and similarly, the nucleotide sequence forming the P10 helix is referred to by the term "P10 region" or "P10 helix region".

In an embodiment of the present disclosure, the nucleotide sequence of the IGS region is 5'-GNNNNN-3', the nucleotide sequence of the target site region is 5'-N'N'N'N'N'U-3', and the P1 helix may be formed by complementarily binding the IGS region and the target site region to each other. In this connection, in order to exclude redundant expressions, the descriptions of the P1 helix region may be used interchangeably to describe the nucleotide sequence of the IGS region.

In the present disclosure, the P1 helix may be formed by including a nucleotide sequence extending in the 5' direction of the IGS region. In this connection, the nucleotide sequence of the 3' direction of the target site facing the extended nucleotide sequence may include a reverse complementary sequence thereof and form an extension of the P1 helix, or may not include a reverse complementary sequence thereof and form a bulge in the P1 helix. However, forming a bulge in the P1 helix is preferable.

In this specification, when the nucleotide sequence extending in the 5' direction of the IGS region exists, the extended nucleotide sequence region is denoted as P1 to distinguish the extended nucleotide sequence region from the IGS region. The same goes for the P10 helix. In this specification, P1 helix variants modified by a nucleotide sequence extending in the 5' direction of the IGS region are referred-to by the terms "P1 extension" and "P1 bulge", respectively. An example of a P1 bulge is illustrated in FIG. 23.

In the present disclosure, the number of bases extending in the 5' direction of the IGS region and in the 3' direction of the target site forming the P1 bulge may each independently be 1 to 10 nt. That is, the length of the extended bases in each region may be the same or different within the above defined numerical range.

Furthermore, in the present disclosure, the nucleotide sequences extending in the 5' direction of the IGS region and in the 3' direction of the target site forming the P1 bulge may be designed not to bind complementarily to each other in whole or in part to form a bulge.

On the other hand, in the present disclosure, the target site region is a region including a base that forms a wobble base pair together with the IGS region, and may be reverse complementary to the IGS region. In addition, in the present disclosure, the target site region may overlap the gene of interest (GOI) region depending on the design of the nucleotide sequence of the IGS region. In other words, in an embodiment of the present disclosure, the target site may overlap part or all of the gene of interest region, or may be separate from the gene of interest region. In this connection, the region that binds complementarily to the IGS region in the gene of interest is separately named "target site" to distinguish that region from the region that binds complimentarily to the IGS region.

In an embodiment of the present disclosure, the AS (antisense sequence) region is located at the 5' end of the self-circularization RNA construct and aims at hydrogen bonding with the nucleotide sequence of the ABS (antisense binding sequence) region located at the 3' end of the RNA construct. Hence, in an embodiment of the present disclosure, the AS region essentially coexists with the ABS region, and accordingly the absence of the AS region may also be understood as including the absence of the ABS region, and likewise, the RNA construct including the AS region may also be understood as including the RNA construct including the ABS region. In the present disclosure, the length of the AS region may affect the efficiency of self-circularization depending on the gene of interest, and so on, and the length of the AS region is indicated by a number following the letter 'AS'. For example, AS150 means an AS region of 150 nt in length.

Meanwhile, the present inventors tried to determine whether the presence or absence of three types of configurations of the P1 region, P10 region, and AS region affects the circularization efficiency achieved by the STS reaction of the self-circularization RNA construct. They identified that an amount of circRNA production was higher in the order of when both the P1 and P10 regions and the AS region were included, when only the P1 region was included, and when only the P1 and P10 regions were included. Additionally, they identified that the self-circularization RNA construct in the case where only the P1 region was included was also circularized with sufficient efficiency to generate circRNA. Accordingly, an embodiment of the present disclosure provides a self-circularization RNA construct in the where only the P1 region is included.

Herein, the self-circularization RNA construct including only the P1 region (P1 construct) means that the P10 region and the AS region do not exist, and does not mean that configurations including regions other than the P10 region and the AS region are excluded. Herein, similarly, the self-circularization RNA construct including only the P1 region and the AS region (P1-AS construct) means that the P10 region does not exist and does not mean that configurations including regions other than the P10 region are excluded.

Hereinafter, embodiments will be described in detail with reference to the accompanying drawings. However, various alterations and modifications may be made to the embodiments. Thus, the scope of the present application shall not be construed as limited or circumscribed by such embodiments. With respect to the embodiments, the scope of the present disclosure includes all changes, equivalents, and replacements.

The terminology used herein is for the purpose of describing particular embodiments only and is not intended to be limiting. The singular forms "a," "an," and "the" used herein include the plural forms as well, unless the context clearly indicates otherwise. The terms "comprises/comprising" and/or "includes/including" used herein, indicate the presence of stated features, integers, steps, operations, elements, and/or components, but do not preclude the presence or addition of one or more other features, integers, steps, operations, elements, components and/or groups thereof.

All terms including technical and scientific terms used herein have the meaning commonly understood by one of ordinary skill in the art to which the examples belong, unless otherwise defined herein. Terms defined in commonly-used dictionaries used herein, should be interpreted as having a meaning that is consistent with the meaning in the context of the relevant art and should not be interpreted in an idealized or overly formal sense unless expressly so defined herein.

In the descriptions of the embodiments making reference to the accompanying drawings, like reference numerals refer to like constituent elements and any repeated description related thereto has been omitted. In the descriptions of the embodiments, any detailed description of well-known related art that was deemed to make the present disclosure ambiguous such that it can be interpreted in more than one way, has been omitted.

### [Examples]

### Example 1. Design of Self-circularization RNA and Production of RNA Expression Vector

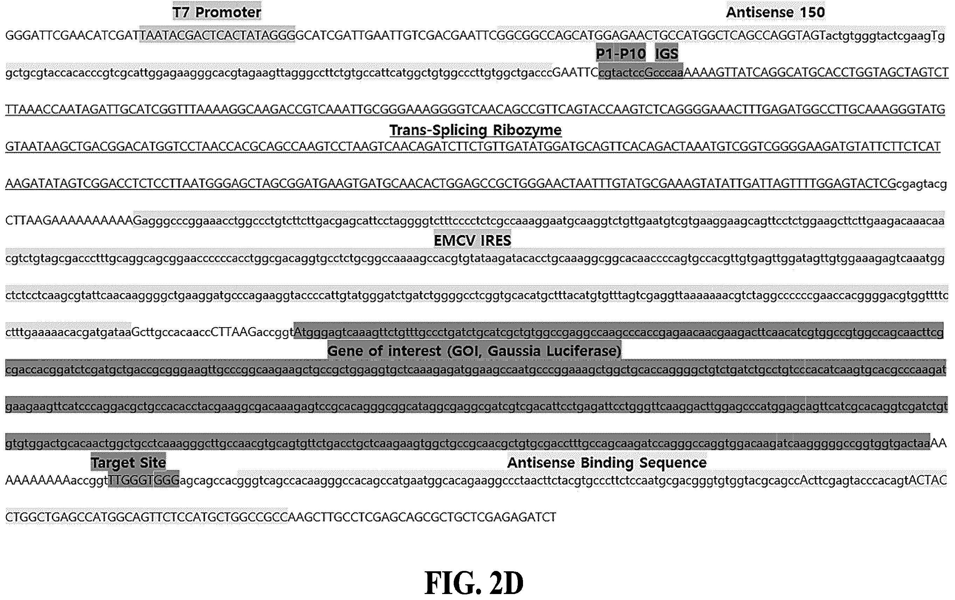

The self-circularization RNA was designed as shown in FIG. 2C, and a T7 promoter sequence was additionally included in the DNA template for its expression to enable in vitro transcription reaction (FIG. 2D). The DNA template was amplified by PCR using a T7 Circular Forward primer: 5'-GGGATTCGAACATCGATTAATACGACTCACTATAGGGGCATCGATTGAATTGT CGA-3' (Tm = 77.5°C) and a T7 Circular Reverse primer: 5'-AGATCTCTCGAGCAGCGCTGCTCGAGGCAAGCTT-3' (Tm = 79.4°C). The DNA template amplification product was inserted into the pTOP TA V2 cloning vector (Enzynomics) using a PstI restriction enzyme to produce a self-circularization RNA expression vector.

### Example 2. Identification of In Vitro Transcription and Self-Circularization

### 2-1. In Vitro Transcription (IVT)

The self-circularization RNA expression vector produced in Example 1 above was transcribed in vitro using a HiScribe T7 high yield RNA synthesis kit (NEB) according to the manufacturer's protocol. Specifically, a 20 uL scale (1 ug T7 DNA template, 1 X Reaction buffer, 10 mM each ATP, UTP, CTP, GTP, T7 RNA polymerase mix 2 ul) was reacted for 3 hours at 37°C, then 29 uL of nuclease-free water was added. Afterwards, 1 uL of RNase-free DNase I (10 U/ul) was added thereto and then the mixture was reacted at 37°C for 30 minutes to induce transcription, after which an immediate circularization (direct STS) reaction was induced.

Subsequently, an additional circularization reaction was induced to identify the stage in which the self-circularization reaction was completed. Specifically, in order to induce the first self-targeting and splicing (1^{st} STS) reaction, 28 uL of nuclease-free water, 20 uL of 5X STS buffer (50 mM Hepes (pH 7.0), 150 mM NaCl, 5 mM MgCl₂), and 2 uL of 100 mM GTP (final 2 mM) were further added to make a volume of 100 uL, after which a self-circularization reaction was performed at 37°C for 1 hour. Then, heating was performed at 55°C for 15 minutes, and after the heating, column purification was performed using a Monarch RNA cleanup kit (NEB). A second STS (2nd STS) reaction was induced by adding 20 uL of 5X STS buffer, 100 mM GTP (final 2 mM), and 28 uL of nuclease-free water to 50 uL of the column-purified sample again to make a final volume of 100 uL and a reaction was performed at 37°C for 3 hours. After the reacting, heating was performed at 55°C for 8 minutes, then column purification was performed using a Monarch RNA cleanup kit (NEB), and the concentration was measured using Nanodrop (Thermo Fisher Scientific product) equipment.

Meanwhile, in order to remove linear RNA from the reaction product, after the first and second STS reactions were performed, some of the column-purified samples were treated with RNase R. Specifically, 10 uL of 10X RNase R reaction buffer (10X: 0.2 M Tris-HCl pH 8.0, 1 M KCl, 1 mM MgCl₂) and 20 units of RNase R were added to column-purified IVT RNA of up to 100 ug (adjusting the volume to 100 uL with water), and the mixture was reacted at 37°C for 30 minutes, and then 10 units of RNase R were further added, and thereby the reaction was performed for another 30 minutes. Then, column purification was performed using a Monarch RNA cleanup kit (NEB) and a concentration was measured with Nanodrop equipment.

250 ng of each of the samples treated with RNase R on RNA obtained from each STS stage and RNA obtained from each stage was mixed in a ratio of 1:1 with 10 M Urea-BPB (IX TBE) dye, heated at 75°C for 5 minutes, and then a 4% polyacrylamide-7 M urea denaturing PAGE (2 hours of electrophoresis at 50 W conditions while maintaining a temperature of 50°C) was performed. Then, the gel was stained with an SYBR Gold Nucleic Acid Stain product (Thermo Fisher Scientific) and analyzed using an ImageQuant 800 (Cytiva product).

As a result, as can be seen in FIG. 3, by the RNase R treatment, RNA bands that were not well cleaved by RNase R and became enriched were revealed (Candidate 1). In addition, bands that were clearly observable despite the RNase R treatment were identified (bands presumed to be Candidate 2 and nicked circular RNA). In addition, it had already been identified that even without additional 1^{st} and 2^{nd} STS reactions, substances presumed to be circRNAs were sufficiently prepared only by a direct STS reaction, that is, an in vitro transcription reaction.

### 2-2. Identification of Self-Circularization

Subsequently, RT-PCR sequencing was performed to verify that a Candidate 1 was a circRNA among the RNA bands not cleaved by RNase R from the PAGE results. Specifically, RT-PCR was performed using a circular RNA sample purified by ethanol precipitation by cutting and crushing the band at the Candidate 1 position in the PAGE results and eluting the band in water at 37°C for 3 hours up to 16 hours and using a linear RNA that does not allow circularization due to the absence of a ribozyme site and an antisense site, as controls, and using primers capable of PCR amplification only when circRNAs were made.

For reverse transcription (RT), OneScript Plus RTase (Abm) was used, and 125 ng of each RNA (a sample of RNAs having or not having been treated with RNase R presumed to be control RNAs and circular RNAs) was heated at 70°C for 5 minutes. Thereafter, the sample was put on ice, and to the sample were added 5X RT buffer 4 uL, 10 mM dNTP mix 1 uL, 2 uM reverse primer (Circular STS R) 1 uL, and OneScript Plus RTase 200 U in an orderly manner. Then, the sample was reacted at 50°C for 15 minutes in a final volume of 20 uL, heated at 95°C for 5 minutes to inactivate the enzyme, and then stored on ice.

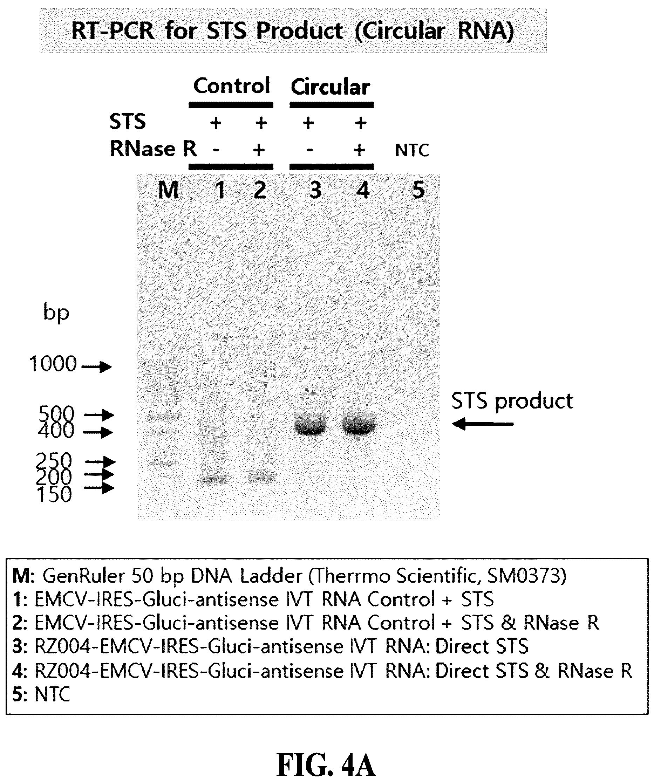

Subsequently, PCR was performed using AccuPower Taq PCR premix (Bioneer), 2 uL of an RT sample and 1 uL each of 20 uM of Circular STS F (5'-CCCTGAGTGGCTGAGCTCAGG-3') and Circular STS R (5'-CAGCAAGCATACTAAATTGCCAG-3') were added and the volume was adjusted to a volume of 20 uL with water. PCR amplification was performed under the conditions of 95°C for 1 minute, [95°C for 30 seconds, 65°C for 30 seconds, and 72°C for 30 seconds], 35 cycles, and 72°C for 5 minutes. 5 uL of a PCR product was put into 1 uL of 6X DNA loading dye, electrophoresed at 150 V for 35 minutes, and images were analyzed using a gel imaging system (Davinch-Gel product of Youngin Scientific). The expected length of the STS PCR product was 479 bp. When analyzed by a GeneRuler 50 bp DNA ladder (Thermo Fisher Scientific) on 1.5% agarose gel (Intron Bio's product, RedSafe Nucleic Acid Staining Solution, is contained in a concentration of 1X), only in the case of an RNA sample presumed to be circular RNA, a specific PCR product of the predicted size was observed regardless of RNase R treatment (FIG. 4A).

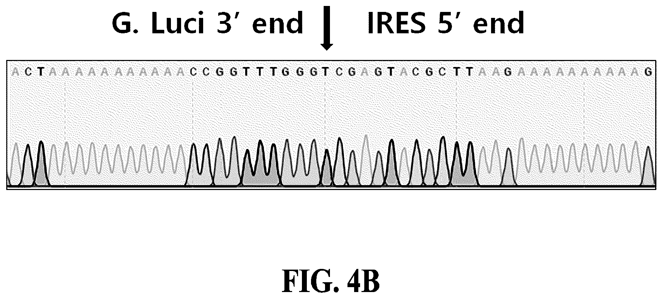

The PCR product was isolated and purified according to the manufacturer's protocol using a gel extraction kit (Cosmogenetech product) from the obtained band of the expected size, cloned using a TOPcloner TA-Blunt kit (Enzynomics product), and transformed into DH5alpha E. coli (Chemically competent E. coli, Enzynomics product) to obtain E. coli colonies from LB-Agar (including Kanamycin) plates. Plasmid DNA was extracted and purified according to the manufacturer's protocol using a DNA purification kit (Cosmogenetech product). Sequencing (Sanger sequencing service of Cosmogenetech, using M13R (-40) or M13F (-20) universal primer provided by the company) was requested. From the sequencing results, it was identified that 3' of Gaussia Luciferase and 5' of IRES were exactly linked at an STS junction site (FIG. 4B).

From the above mentioned results, it can be seen that a Candidate 1 is a circular RNA.

### 2-3. Revalidation of Self-Circularization

It was identified that a Candidate 1 is a circRNA through RT-PCR, but it was determined that theoretically, the possibility that the Candidate 1 can be in the form of a dimer may not be excluded. Accordingly, a nicking test was performed to revalidate that a Candidate 1 is a circRNA.

MgCl₂ was mixed with purified circular RNA Candidates 1 and 2 (100 ng) to achieve final concentrations of 0, 2.5, and 5 mM, respectively. The sample present in the final 10 uL of water was heated at 65°C for 30 minutes and then stored on ice for a while. Afterwards, the sample was mixed with 10 uL of 10 M Urea-BPB (IX TEB) loading dye.

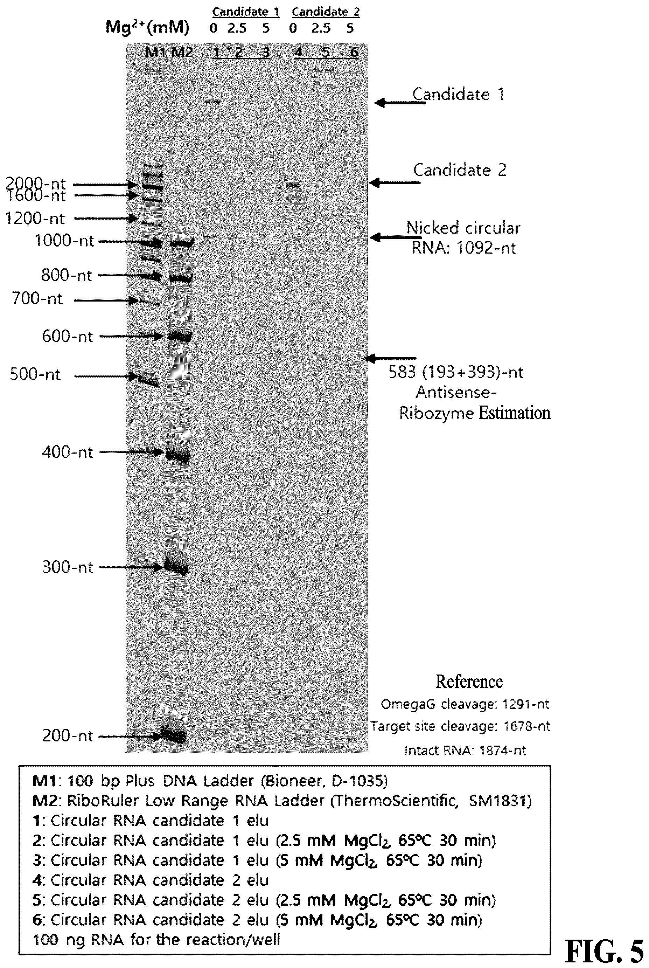

The mixture was heated at 75°C for 5 minutes. After the heating, 4% polyacrylamide-7 M urea denaturing PAGE (2 hours of electrophoresis at 50 W conditions while maintaining a temperature of 50°C) was performed. Gels were stained with an SYBR Gold Nucleic Acid Stain product (Thermo Fisher Scientific) and analyzed using an ImageQuant 800 (Cytiva product). The results are shown in FIG. 5.

For Candidate 1, it was identified that in the absence of Mg²⁺, a band with a size corresponding to a slightly nicked circular RNA was included (1092-nt), and in the case of a 2.5 mM Mg²⁺ condition, the band at the position of Candidate 1, which is believed to be the position of a circular RNA, was decreased. In addition, it was identified that a band of nicked circular RNA size was still present. In the case of a 5 mM Mg²⁺ condition, it was identified by hydrolysis that even the nicked circular RNA band disappeared. Thus, it was observed that Candidate 1, that is, circular RNA, started at the size of 1092-nt as expected for a monomer when nicking occurred (2.5 mM Mg²⁺) and eventually the Candidate 1 completely degraded (5 mM Mg²⁺). Accordingly, it was identified again that the Candidate 1 is a circular RNA and a monomer.

On the other hand, the size of Candidate 2 is similar to the size of intact RNA, 1874-nt (around 2000-nt of the marker). Unlike Candidate 1, under mild nicking conditions of 2.5 mM Mg²⁺, it was identified that the band of 1092-nt, the size of the nicked circular RNA, was not generated and disappeared, and thus that Candidate 2 was not a circular RNA.

### Example 3. Identification of Intracellular Transcription and Self-Circularization

In Example 2, it was identified that the self-circularization RNA expression vector produced in Example 1 was transcribed in vitro to form a circRNA. Accordingly, the intention was to identify whether the vector operated in the same way in cells, and furthermore, whether the gene of interest included in the circRNA was expressed smoothly in the cells.

For expressing a gene of interest in cells, the gene of interest was designed with the structure of 5'-EMCV IRES-transgene-stop codon-3', and so the expressed gene of interest could be easily identified, gaussia luciferase (G.luci) was used as a transgene.

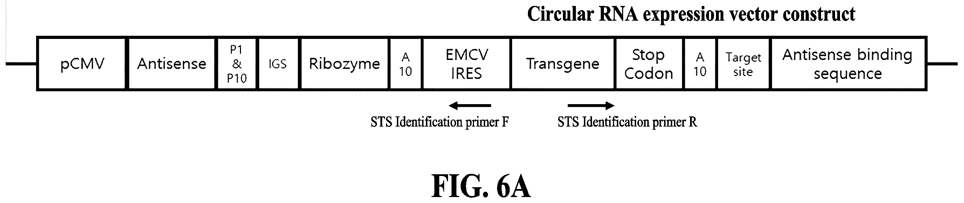

A self-circularization RNA construct including the aforementioned gene of interest was designed, and a DNA template, which may express the same, was inserted into a plasmid to be expressed under a pCMV promoter (FIG. 6A). The plasmid vector was transfected into 293A cells, the generation of a circRNA was identified by RT-PCR and sequencing, and the expression of transgene was identified by performing a luciferase activity assay.

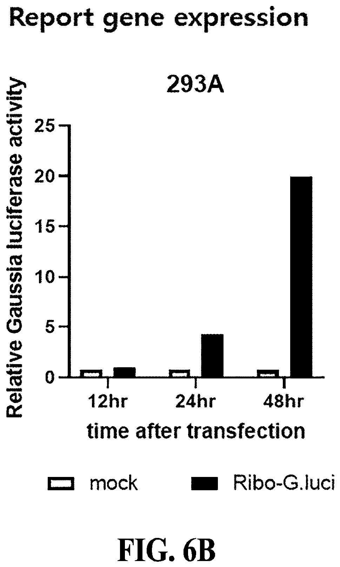

Specifically, 293A cells were seeded at 2×10⁵/well in 6-well plates and after 24 hours were transformed into the plasmid vector using a lipofectamine 2000 transfection reagent. After 6 hours of transformation, a culture medium was replaced. After the transformation, 100 UL of culture medium was collected at 12, 24, and 48 hours to measure G.luci activity. It was identified that G.luci activity was detected in the culture medium and the activity increased over time, showing that the transgene G.luci gene was expressed in the vector introduced into the cells (FIG. 6B).

It was investigated at the molecular level through RT-PCR and sequencing whether transgene expression was caused by the formation of a circRNA. 48 hours after transformation, total RNA was extracted from the 293A cells introduced with the plasmid vector using a trizol reagent, and then RT-PCT was performed, through which it was identified that circular RNA was generated.

For reverse transcription (RT), OneScript Plus RTase (Abm product) was used, and 1 ug of total RNA was heated at 70°C for 5 minutes and then placed on ice. Thereafter, 5X RT buffer 4 uL, 10 mM dNTP mix 1 uL, 2 uM reverse primer (Circular STS R) 1 uL, and OneScript Plus RTase 200 U were added thereto in an orderly manner. Then, the mixed sample was reacted at 50°C for 15 minutes in a final volume of 20 uL, heated at 95°C for 5 minutes to inactivate the enzyme, and then stored on ice.

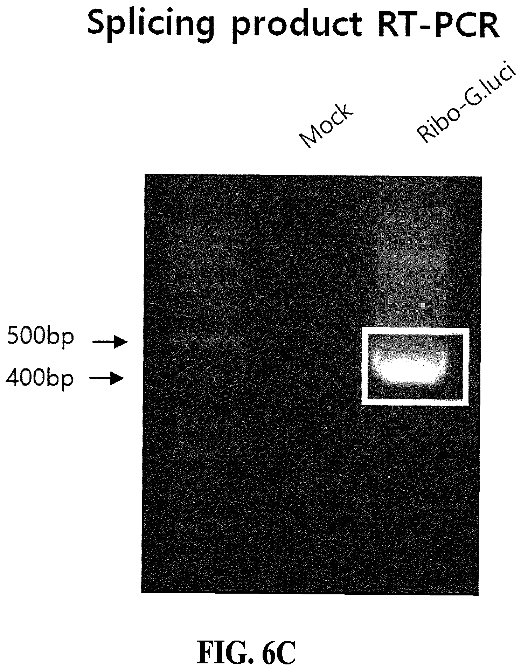

Subsequently, PCR was performed using AccuPower Taq PCR premix (Bioneer product), 2 uL of an RT sample and 1 uL each of 20 uM of Circular STS primer F (5' - caaggacttggagcccatggagcag - 3') and primer R (5' - tgtgccgcctttgcaggtgtatc - 3') were added, the volume was adjusted to a volume of 20 uL with water, and PCR amplification was performed under the conditions of 95°C for 1 minute, [95°C for 30 seconds, 65°C for 30 seconds, and 72°C for 30 seconds] 35 cycles, and 72°C for 5 minutes. The expected length of the STS PCR product was 479 bp. When analyzed by a GeneRuler 50 bp DNA ladder (Thermo Fisher Scientific product) on 1.5% agarose gel (Intron Bio product, RedSafe Nucleic Acid Staining Solution, is contained in a concentration of 1X), a specific PCR product of the predicted size was identified only when circular RNA was present. In this connection, 5 uL of a PCR product was put into 1 uL of 6X DNA loading dye, the product was electrophoresed at 150 V for 35 minutes, and images were analyzed using a gel imaging system (Davinch-Gel product of Youngin Scientific) (FIG. 6C).

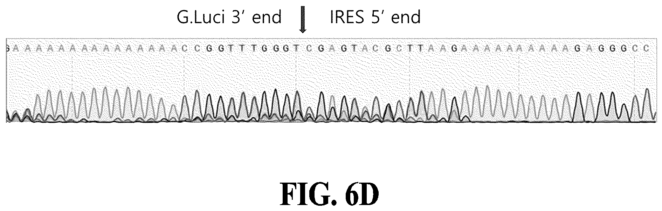

In addition, the PCR product was isolated and purified according to the manufacturer's protocol using a gel extraction kit (Cosmogenetech product) from the obtained band of the expected size, cloned using a TOPcloner TA-Blunt kit (Enzynomics product), and transformed into DH5alpha E. coli (Chemically competent E. coli, Enzynomics product) to obtain E. coli colonies from LB-Agar (including Kanamycin) plates. Plasmid DNA was extracted and purified according to the manufacturer's protocol using a DNA purification kit (Cosmogenetech product) and manual. Sequencing (Sanger sequencing service of Cosmogenetech, using M13R (-40) or M13F (-20) universal primer provided by the company), was requested. From the sequencing results it was identified that 3' of Gaussia Luciferase and 5' of IRES were exactly linked nucleotide sequences at an STS junction site (FIG. 6D).

From the above, it was identified at the molecular level that the produced plasmid vector expressed the self-circularization RNA construct in the cells, the construct was circularized into a circRNA by a ribozyme, and the gene of interest was expressed in the circRNA.

### Example 4. Purification of circRNA

### 4-1. Identification of circRNA Purification Potential by Performing HPLC

In order to minimize the occurrence of immunogenicity in the production of a circRNA for human injection, analysis and purification using HPLC were performed. Specifically, an Agilent 1290 Infinity II Bio UHPLC system was used. The analysis conditions are shown in FIG. 7A. For the analysis, the gradient conditions of [Analytical] were used, and for sample fractionation the conditions of [Fraction collection] were used.

When RNA purified only with a column (a Monarch RNA cleanup kit (NEB)) after IVT (FIG. 7B) and RNA treated with RNase R (FIG. 7C) were analyzed, an increasing peak in the RNase R-treated sample was found, indicating that a circRNA could be isolated by HPLC without RNase R treatment.

### 4-2. Purification of circRNA by Performing HPLC

Through HPLC, fractionation was performed using the gradient shown in the [Fraction collection] conditions, and each fraction was obtained as shown in FIG. 8A. In the same manner as before, 4% denaturing PAGE was performed by loading 200 ng each of fractions 7 and 10 to 13 with clear peaks.

As a result, as can be seen in FIG. 8B, clean circular RNA was isolated and purified from fraction 12.

### Example 5. Antisense Sequence (AS) Region Optimization

The intention was to identify the effect of the lengths of an antisense sequence (AS) region and a reverse-complementary antisense binding sequence (ABS) region on the immediate circularization reaction during an in vitro transcription process. Accordingly, a DNA template having different lengths of the AS region and ABS region of 50, 100, 150, 200, 250, or 300-nt was prepared (FIG. 9). A vector capable of expressing each RNA construct was produced in a manner like the one used for Example 1, and in vitro transcription was performed at 37°C for 3 hours in a manner like the one used for Example 2. The degree of immediate STS reaction was compared and identified through relative band intensity by performing PAGE on 4% polyacrylamide-7 M urea gel (20×20 cm, 1 mm).

The nucleotide sequences of each AS region are shown in Table 1 below.

**[Table 1]**

| Sections | Nucleotide sequences (5' --> 3') |
|---|---|
| AS50 | AAGTTAGGGCCTTCTGTGCCATTCATGGCTGTGGCCCTTGTGGCTGACCC |
| AS100 | ACTCGAAGTGGCTGCGTACCACACCCGTCGCATTGGAGAAGGGCACGT AGAAGTTAGGGCCTTCTGTGCCATTCATGGCTGTGGCCCTTGTGGCTGA CCC |
| AS150 | GGCGGCCAGCATGGAGAACTGCCATGGCTCAGCCAGGTAGTACTGTGG GTACTCGAAGTGGCTGCGTACCACACCCGTCGCATTGGAGAAGGGCAC GTAGAAGTTAGGGCCTTCTGTGCCATTCATGGCTGTGGCCCTTGTGGCT GACCC |
| AS200 | AGCGTGAGGAAGTTGATGGGGAAGCCCAGCACGATCAGCAGAAACATG TAGGCGGCCAGCATGGAGAACTGCCATGGCTCAGCCAGGTAGTACTGT GGGTACTCGAAGTGGCTGCGTACCACACCCGTCGCATTGGAGAAGGGC ACGTAGAAGTTAGGGCCTTCTGTGCCATTCATGGCTGTGGCCCTTGTGG CTGACCC |
| AS250 | GGATGTAGTTGAGAGGCGTGCGCAGCTTCTTGTGCTGGACGGTGACGTA GAGCGTGAGGAAGTTGATGGGGAAGCCCAGCACGATCAGCAGAAACAT GTAGGCGGCCAGCATGGAGAACTGCCATGGCTCAGCCAGGTAGTACTG TGGGTACTCGAAGTGGCTGCGTACCACACCCGTCGCATTGGAGAAGGGC ACGTAGAAGTTAGGGCCTTCTGTGCCATTCATGGCTGTGGCCCTTGTGG |
| | CTGACCC |
| AS300 | GGTGAAGCCACCTAGGACCATGAAGAGGTCAGCCACGGCTAGGTTGAG CAGGATGTAGTTGAGAGGCGTGCGCAGCTTCTTGTGCTGGACGGTGACG TAGAGCGTGAGGAAGTTGATGGGGAAGCCCAGCACGATCAGCAGAAAC ATGTAGGCGGCCAGCATGGAGAACTGCCATGGCTCAGCCAGGTAGTAC TGTGGGTACTCGAAGTGGCTGCGTACCACACCCGTCGCATTGGAGAAGG GCACGTAGAAGTTAGGGCCTTCTGTGCCATTCATGGCTGTGGCCCTTGT GGCTGACCC |

As a result, as can be seen in FIG. 10 and Table 2 below, in the case of AS50, AS100, and AS150, the self-circularization efficiency was relatively excellent compared to the length of AS200 or longer. On the other hand, for AS50 and AS 100, total RNA produced after the in vitro transcription reaction was significantly less. Accordingly, it was found that AS150 was the most optimal length for self-circularization RNA construct expression and circular RNA production.

**[Table 2]**

| AS series | AS50 | AS100 | AS150 | AS200 | AS250 | AS300 |
|---|---|---|---|---|---|---|
| A: Amount of Total RNA produced (µg) | 69.5 ± 1.77 | 9.15 ± 0.46 | 176.5 ± 3.89 | 140 ± 3.54 | 133 ± 2.83 | 141 ± 5.66 |
| B: Relative band intensity of circular RNA (%) | 6.35 ± 0.25 | 6.75 ± 0.04 | 5.95 ± 0.18 | 4.30 ± 0.07 | 4.20 ± 0.07 | 3.60 ± 0.14 |
| Relative factor (A * B) | 441 | 62 | 1050 | 602 | 559 | 508 |

### Example 6. Spacer Region Optimization

Subsequently, in order to identify the effect of the length or type of the spacer region on the immediate circularization reaction during an in vitro transcription process, a DNA template including spacer regions of different lengths and nucleotide sequences was produced (FIG. 11). A vector capable of expressing each RNA construct was produced in a manner like the one used for Example 1, and in vitro transcription was performed at 37°C for 3 hours in a manner like the one used for Example 2. The degree of immediate STS reaction was compared and identified through relative band intensity by performing PAGE on 4% polyacrylamide-7 M urea gel (20×20 cm, 1 mm). The nucleotide sequences of each spacer region are shown in Table 3 below. In this experiment, the spacers of A10, A30, and A50 were used by adding a restriction site at the 3' end for IRES insertion immediately after the spacer region. In this experiment, the AatII site (GACGTC) was added to the 3' end of the spacer and used.

**[Table 3]**

| Sections | Nucleotide sequences (5' --> 3') |
|---|---|
| A10 | AAAAAAAAAA |
| A30 | AAAAAAAAAAAAAAAAAAAAAAAAAAAAAA |
| A50 | AAAAAAAAAAAAAAAAAAAAAAAAAAAAAAAAAAAAAAAA AAAAAAAAAA |
| Control spacer 1 | GGTAGTGGTGCTACTAACTTCAGCCTGCTGAAGCA |
| Control spacer 2 | GGTAGTAAACTACTAACTACAACCTGCTGAAGCA |

As a result, as can be seen in FIG. 12 and Table 4 below, despite the difference in the length and sequence of the spacer, it was found that there was no significant difference in the immediate circularization efficiency during the in vitro transcription process, but that A30 operated as the most optimal spacer for self-circularization RNA construct expression and circular RNA production.

**[Table 4]**

| AS150 | A10 | Control spacer 1 | Control spacer 1 | A30 | A50 |
|---|---|---|---|---|---|
| Spacer version | | | | | |
| A: Amount of Total RNA produced (µg) | 199.5 ± 9.19 | 199.5 ± 6.36 | 187 ± 5.66 | 190 ± 8.49 | 192.5 ± 0.71 |
| B: Relative band intensity of circular RNA (%) | 5.55 ± 0.35 | 6.7 ± 0.14 | 6.15 ± 0.07 | 6.5 ± 1.27 | 5.7 ± 0.85 |
| Relative factor (A * B) | 1106 ± 19 | 1311 ± 70 | 1150 ± 21 | 1240 ± 297 | 1097 ± 160 |

### Example 7. Optimization of Self-circularization RNA Construct

### 7-1. AS Region, and P1 Helix and P10 Helix Regions

The self-circularization RNA construct designed in Example 1 includes an AS region, and P1 helix and P10 helix regions. In order to identify the effect of each configuration on the immediate circularization reaction during the in vitro transcription process, as illustrated in FIG. 13, a DNA template including only the P1 helix region, only the P1 and P10 helix regions, or both the P1 and P10 helixes and the AS region was prepared. A vector capable of expressing each RNA construct was produced in a manner like the one used for Example 1, and in vitro transcription was performed at 37°C for 3 hours in a manner like the one used for Example 2. The degree of immediate STS reaction was compared and identified through relative band intensity by performing PAGE on 4% polyacrylamide-7 M urea gel (20×20 cm, 1 mm).

The nucleotide sequences of the T7 DNA template including only the P1 helix region are shown in FIG. 14.

As a result, as can be seen in FIG. 15 and Table 5 below, it was found that there was no significant difference in the total RNA transcribed and generated in vitro for each vector, but that the amount of circular RNA produced was higher in the order of the case where both the P1 and P10 helixes and the AS region were included, the case where only the P1 helix region was included, and the case where only the P1 and P10 regions were included.

**[Table 5]**

| AS150 | P1 (No AS) | P1&P10 (No AS) | P1&P10 (AS150) |
|---|---|---|---|
| A: Amount of Total RNA produced (µg) | 200 | 196 | 182 |
| B: Relative band intensity of circular RNA(%) | 3.2 | 0.5 | 5.1 |
| Relative factor (A * B) | 640 | 98 | 928 |

7-2. Circularization Verification of Self-circularization RNA Construct that Does Not Include P10 and AS Regions

From the results of Example 7-1, it was found that sufficient circRNAs could be produced without an additional circularization stage during the in vitro transcription process from a DNA template (P1 construct) that does not include P10 and AS regions. In order to verify this finding, the product after the STS reaction of Example 7-1 was treated with RNase R to remove linear RNA, PAGE was performed in a manner like the one used for the previous experiment, and then RT-PCR and sequencing were performed in a manner like the one used for Example 2-2.

As a result, as can be seen in FIGS. 16A and 16B, even when self-circularization was performed using the P1 construct, upon treatment with RNase R, an RNA band that was not easily cut by RNase R and enriched was observed. As a result of performing RT-PCR and sequencing on the STS reaction product in the band, it was identified that the product was a circRNA.

### Example 8. Optimization of P1 Helix Region

### 8-1. P1 Helix Region and Self-Circularization

From the results of Example 7, it was identified that the self-circularization construct without P10, AS, and ABS is sufficient to make a circRNA. Accordingly, the intention was to verify assuming that a circular RNA would be generated if only the Internal Guide Sequence (IGS) present at the 5' end and the nucleotide sequence of the target site present at the 3' end constituting the P1 helix are complementarily bound to each other (U and G are wobble base pairs).

Since the IGS region is GNNNNN and the nucleotide sequence of the target site is N'N'N'N'N'U, when only one U nucleotide is added to the GOI, the finally generated circRNA consists only of one additional U base and the GOI region (FIGS. 1A and 1B). In addition, when the 3' end of the GOI ends with a U base, by designing the nucleotide sequence of the IGS region to be reverse-complementary to the GOI, the circRNA to be finally generated may be prepared such that it consists only of the GOI region (FIGS. 20A and 20B).

As shown in FIG. 17, various sequences of IGS and target site were designed, a vector was produced by the method of Example 1, and then the in vitro transcription (IVT) of Example 2-1 was performed.

As a result, as can be seen in FIGS. 18-19 and Table 6 below, it was identified that when both ends have complementary sequences, there is an increase in the amount of circRNA generated through the immediate STS reaction after in vitro transcription. In particular, it was identified that the efficiency of circRNA production was higher when the sequences of IGS and target site were AU-rich sequences. On the other hand, it was found that the vector including the no-complement IGS region generated a very low amount of circRNA. From the above, it was found that both the IGS and the target site can efficiently induce a self-circularization reaction when they are complementary to each other.

**[Table 6]**

| P1 variants (No AS) | AS150 (P1&P10) | RZ004 | RZ001 | RZ003 | GC-rich | AU-rich | 2 sites | No complement |
|---|---|---|---|---|---|---|---|---|
| Relative band intensity of circular RNA(%) | 5.4 | 3.7 | 7.9 | 6.6 | 5.4 | **11.6** | 6.1 | 1 |

### Example 9. Modification of P1 Helix Region

Through the experiments of Example 7, it was confirmed that the P10 region reduces the efficiency of circular RNA production in the absence of the AS region, and through the experiments of Example 8, it was identified that when the IGS and the target site forming the P1 helix are designed with reverse complementary sequences, self-circularization reaction may be efficiently induced.

Next, the present inventors modified the P1 helix region by adding bases in the 5' direction from the 5'-GNNNNN-3' of the IGS and in the 3' direction from the 5'-N'N'N'N'N'U-3' of the target site region to determine whether the modification affected the efficiency of circular RNA production. Specifically, the effect on the efficiency of circular RNA production was investigated by designing the bases extending in the 5' direction of the IGS region and the 3' direction of the target site region to be reverse complementary to each other to extend the length of the P1 helix (hereinafter, referred to by "P1 extension"), or designing the extended bases such that they do not bind complementarily to each other to form a P1 helix including a bulge (hereinafter, referred to by "P1 bulge").

Accordingly, as shown in FIG. 21A, bases were added in the 5' direction of the IGS region to design the P1 extension, P1 bulge + AS construct, and P1 extension & bulge AS construct. To produce the vector for expression of each of the above-mentioned constructs, each gene fragment was first synthesized with gBlock and then inserted into the In-fusion HD cloning kit (TAKARA) using restriction enzymes (PstI, NheI, Pml, and SpeI) to produce the expression vectors (FIG. 21B). Each of the above-mentioned construct expression vectors was PCR amplified using the primers shown in FIG. 21C. Subsequently, using the expression vectors for each of the above-mentioned constructs, the in vitro transcription and direct STS reaction of Example 2-1 were induced, and then the column purification of Example 2-1 was performed without RNase R treatment having been performed, and the concentration was measured using Nanodrop equipment. 250 ng of each sample was mixed in a ratio of 1:1 with 10 M Urea-BPB (IX TBE) dye, heated at 75°C for 5 minutes, and then a 4% polyacrylamide-7 M urea denaturing PAGE (2 hours of electrophoresis at 50 W conditions while the temperature was maintained at 50°C) was performed. Then, the gel was stained with an SYBR Gold Nucleic Acid Stain product (Thermo Fisher Scientific) and analyzed using an ImageQuant 800 (Cytiva product).

As a result, as shown in FIG. 22A, circular RNA production rate was higher for the P1 bulge-AS150 construct than the P1 construct, and further, circular RNA production efficiency was higher for the P1 bulge-AS150 construct than the P1-P10-AS150 construct. On the other hand, it appears that extending the P1 helix did not affect the self-circularization efficiency of the construct. The P1 extension and P1 extension-bulge-AS150 constructs did not show a tendency that would indicate progress toward meaningful improvement in self-circularization efficiency.

Meanwhile, in order to re-confirm the high circular RNA production result for the P1 bulge-AS150 construct mentioned above, some of the column-purified samples were treated with RNase R and subjected to PAGE to remove linear RNA from the reaction products after the in vitro transcription and direct STS reaction induction, in a manner like the one used for Example 2-1 (FIG. 22B). Then, the band at the circRNA location was selected from the results of the above mentioned PAGE, and RT-PCR of Example 2-2 was performed (FIG. 22C). Then, sequencing was performed (FIG. 22D). From the above mentioned results, it was confirmed that the P1 bulge-AS150 construct formed circular RNA after a direct STS reaction was performed under IVT conditions.

Although a number of embodiments have been described with reference to limited drawings, one of ordinary skill in the art will recognize that various modifications and alterations may be made to these embodiments based on the above detailed description. For example, suitable results may be achieved if the described techniques are performed in a different order, and/or if components in a described system, architecture, device, or circuit are combined in a different manner, and/or replaced or supplemented by other components or their equivalents.

Therefore, other implementations, other examples, and equivalents to the claims are also within the scope of the following claims.

### [Industrial Applicability]

The present disclosure may be used to produce circRNAs for protein expression in vitro, in cells, and in vivo, and may be used to produce functional RNA such as miRNA, anti-miRNA, shRNA, aptamers, mRNA vaccines, mRNA therapeutic agents, antibodies, vaccine adjuvants, and CAR-T mRNA as a circRNA.

## Claims

1. A self-circularization RNA construct, wherein:
the construct has a structure of 5' - IGS (internal guide sequence) - ribozyme - gene of interest - target site - 3';
the IGS region comprises 5'-GNNNNN-3' and the target site comprises 5'-N'N'N'N'N'U-3', so that the guanine (G) of the IGS region and the uracil (U) of the target site form a wobble base pair;
the nucleotide sequence of 5'-GNNNNN-3' of the IGS region comprises a sequence that is reverse complementary to the nucleotide sequence of the target site region, except for the guanine, forming a secondary structure (P1 helix);
the IGS region comprises a nucleotide sequence extending in the 5' direction of the 5'-GNNNNN-3';
the target site comprises a nucleotide sequence extending in the 3' direction of the 5'-N'N'N'N'N'U-3';
regions of the nucleotide sequences extending from the IGS region and the target site region form a bulge in the P1 helix.

2. The self-circularization RNA construct of claim 1, wherein the target site region overlaps a gene of interest region.

3. The self-circularization RNA construct of claim 1, wherein the length of each nucleotide sequence extending in the 5' direction of the IGS region and in the 3' direction of the target site forming the bulge in the P1 helix is independently 1 to 10 nt.

4. The self-circularization RNA construct of claim 1, wherein the ribozyme is a Group I intron ribozyme.

5. The self-circularization RNA construct of claim 1, wherein the ribozyme comprises a nucleotide sequence represented by SEQ ID NO: 6.

6. The self-circularization RNA construct of claim 1, wherein the construct does not form a P10 helix.

7. The self-circularization RNA construct of claim 1, wherein the construct further comprises an antisense sequence (AS) region in the 5' direction of the IGS region and an antisense binding sequence (ABS) region capable of complementary binding to the AS region in the 3' direction of the target site.

8. The self-circularization RNA construct of claim 7, wherein a length of the AS region is 50-nt to 400-nt.

9. The self-circularization RNA construct of claim 1, wherein the gene of interest region comprises an internal ribosome entry site (IRES) region at the 5' end.

10. The self-circularization RNA construct of claim 1, wherein the construct has a ribozyme region and a gene of interest region linked by a spacer region comprising a random nucleotide sequence.

11. The self-circularization RNA construct of claim 1, wherein the construct has a gene of interest region and a target site region linked by a spacer region comprising a random nucleotide sequence.

12. A vector expressing the self-circularization RNA construct of claim 1.

13. The vector of claim 12, wherein the vector comprises a promoter operably linked to a gene encoding a self-circularization RNA.
